# EUROPEAN PATENT APPLICATION

(11) **EP 2 544 112 A1**
(43) Date of publication of application: **09.01.2013**
(21) Application number: 12179946.4
(22) Date of filing: 20.11.2007
(51) Int. Cl.: G06F 19/00

(54) **System and method for remote monitoring and/or management of infusion therapies**

(30) Priority: 21.11.2006 US 860597 P
(62) Divisional of application: 07864680.9
(71) Applicant: Baxter International Inc., Deerfield, Illinois 60015 (US); Baxter Healthcare S.A., 8152 Glattpark (Opfikon) (CH)
(72) Inventor: Moubayed, Ahamd-Maher, Mission Viejo, CA 92692 (US); Hyman, Oscar E., Poulsbo, WA 98370 (US); White, David N., San Juan Capistrano, CA 92675 (US); Wilson, Larry L., Poway, CA 92064 (US); Stevenson, John W., Carlsbad, CA 92010 (US); Moubayed, Jay G., Aliso Viejo, CA 92656 (US); Thomas, Linda, Laguna Niguel, CA 92677 (US)
(74) Representative: Dee, Ian Mark

(57) **Abstract**

A system and method for remote monitoring and/or management of infusion therapies. A user can monitor and manage server-connected pumps at a remote location, such as a computer or PDA. Pumps located at an institution, such as a hospital or patients home, are connected, for example, via the Internet to a server that includes a database of information. A user can operate the pump, from a remote location, by using an interface displayed on the remote site. The operator can manage pump operations by use of the interface. In this context, the user can turn the pump on and off, select infusion rates, dose amounts, etc. all from the convenience of the remote location.

## Description

### CLAIM OF PRIORITY

This application claims priority under 35 USC 120 to U.S. Provisional Application Serial No. 60/860,597, filed November 21, 2006, the contents of which are hereby incorporated by reference.

### TECHNICAL FIELD

This application relates to a system and method for monitoring and managing infusion therapy, and in particular, from a remote location.

### BACKGROUND

Infusion pumps are used in the field of health care are electromechanical devices which control the flow rate of medical fluids. These pumps operate, for example, to deliver a drug to a patient at a precisely controlled rate. A vital application for infusing pumps in the human and veterinary medical field is in the delivery of chemicals, drugs, nutrition, or biological products to patients. Typically, one or more drugs or other substances are mixed into a uniform solution in a medical fluid and are then delivered through an infusion pump into the bloodstream of a patient via tubing and/or catheters which conduct the fluid from the pump to the patient's vascular space. The fluid rate or sequence of rates at which an infusion pump operates is typically selected based on desired pattern of drug delivery appropriate to the specific circumstance. Numerous factors should be considered when specifying a specific rate, amount, etc. of fluid to flow from a pump at any given time.

Prior art pumps to deliver fluid include various infusion pump systems having varying degrees of programmability and/or automation. Examples of infusion pump systems that are programmable and/or have some degree of automation include, but are not limited to, those described in United States Patent Nos. 4,670,007 (Wheeldon et al.); 4,978,335(Arthur, III); 4,976,151 (Morshita); 4,856,339 (Williams); 5,256,157 (Samiotes, et al.); 5,756,327 (Sasanfar, et al.); 5,683,367 (Jordan, et al.); 6,269,340 (Ford, et al.); 6,854,620 (Ramey) and 6,659,980 (Moberg, et al.) as well as United States Patent Application Publication Nos. 2004/0019607 (Moubayed et al.) and 2004/0172283 (Vanderveen et al.).

Additionally, at least one commercially available infusion system exists that includes a mobile systems manager which communicates by wireless connection to one or more central server(s) and various individual infusion pumps located within range of the wireless network (Alaris Mobile Systems Manager, Cardinal Health, Inc., San Diego, California).

With heightened emphasis on cost-effectiveness and cost-containment in health care, clinic and home infusion therapy is becoming increasingly commonplace. For example, home infusion therapy generally involves the administration of medications, for example, immune globulin infusions using intravenous, or subcutaneous routes, in the patient's home rather than in a physician's office or hospital. Infusion therapies in the home are typically administered by a home health care worker having some degree of training in the operation of infusion equipment and the administration of biologic therapies. In some cases, the patient him/herself administers the therapy.

### SUMMARY

A system and method for remote monitoring and/or management of infusion therapies. A user can monitor and manage server-connected pumps at a remote location, such as a computer or PDA. Pumps located at an institution, such as a hospital or patients home, are connected, for example, via the Internet to a server that includes a database of information. A user can operate the pump, from a remote location, by using an interface displayed at the remote location. The operator can manage pump operations by use of the interface. In this context, the user can turn the pump on and off, select infusion rates, dose amounts, etc. all from the convenience of the remote location.

### BRIEF DESCRIPTION OF THE DRAWINGS

Figure 1 illustrates an exemplary system in accordance with the invention.

Figure 2 shows an exemplary navigation screen.

Figure 3 shows an exemplary scrollable text window to display pump history content.

Figure 4 shows an exemplary remote monitoring page.

Figure 5 shows another exemplary remote monitoring options page.

Figure 6 shows an exemplary Rx Access/Programming page which allows an operator to select an exiting Rx program.

Figure 7 shows an exemplary IVIG Rx Programming page.

Figure 8 shows an exemplary SCIG Rx Programming page.

Figure 9 shows a CONTINUOUS Rx Programming page.

Figure 10 shows an exemplary INTERMITTENT Rx Programming page.

Figure 11 shows a TPN Rx Programming page.

Figure 12 shows an exemplary IVIG Drug page.

Figure 13 shows an exemplary SCIG/CONT/INT/TPN Drug page.

Figure 14 shows an exemplary patient record maintenance page.

Figure 15 shows an exemplary patient delete page.

Figure 16 shows an exemplary new patient page.

Figure 17 shows an exemplary Rx record maintenance page.

Figure 18 shows an exemplary diagram of an Rx Save Conflict page.

Figure 19 shows an exemplary Drug Reference Library (DRL) maintenance page.

Figure 20 shows an exemplary IVIG Drug Reference Record (DRR) page / Rx Validation tab page.

Figure 21 shows an exemplary SCIG DRR page / Rx Validation tab page.

Figure 22 shows an exemplary CONTINUOUS DRR page / Rx Validation tab page.

Figure 23 shows an exemplary INTERMITTENT DRR page / Rx Validation tab page.

Figure 24 shows an exemplary DRR Vital sign tab page.

Figure 25 shows an exemplary DRR Symptoms tab page.

Figure 26 shows an exemplary DRR Frequency tab page.

Figure 27 shows an exemplary DRR Pre-Infusion Check List tab page.

Figure 28 shows an exemplary DRR Applicable tab page.

Figure 29 shows an exemplary DRR Notes tab page.

Figure 30 shows an exemplary DRR Add/Delete Symptoms page.

Figure 31 shows an exemplary DRR ARM Function tab page.

Figure 32 shows a Configure SCC Users page.

Figure 33 shows an exemplary Add/Edit User page.

Figure 34 shows an exemplary Administrator Configuration Parameters (ACP) Maintenance page.

Figures 35-41 show an exemplary ACP Global, Users, IVIG, SCIG, CONT, INT and TPN tab pages.

Figure 42 shows an exemplary Update Pump DRL and/or ACP page.

### DETAILED DESCRIPTION

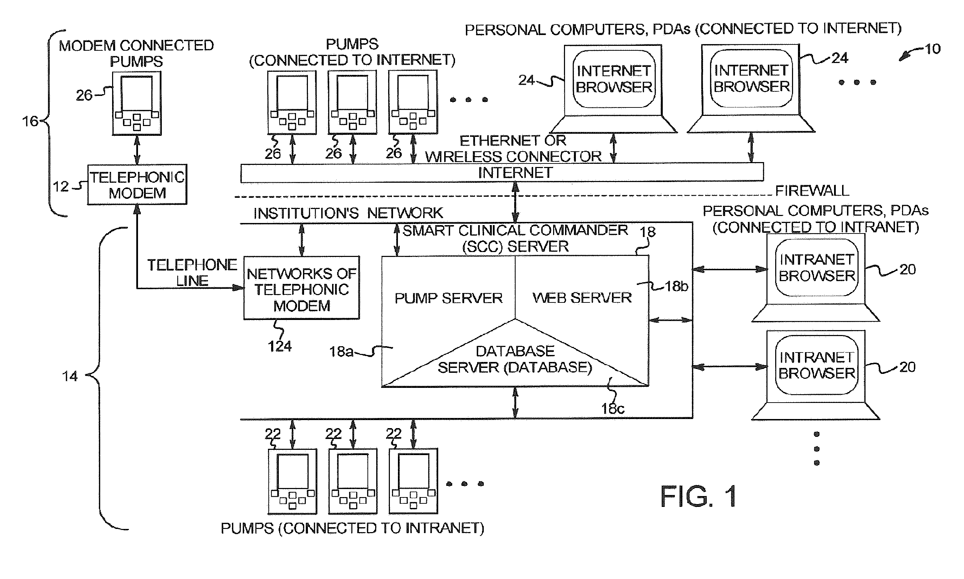

The present disclosure provides a system and method wherein one or more remotely located user interface devices (e.g., personal computers, personal digital assistants, etc.) are connected, via the Internet and/or directly into an institutions hard-wired or wireless network, to server(s) and infusion pumps located within an institution (e.g., hospital) or at remote locations (e.g., patient's homes). One embodiment or example of a system of the present disclosure is shown schematically in Figure 1.

As shown in Figure 1, the system 10 includes an intra-institutional portion 14 located within an institution (e.g., a hospital, home health agency, clinic, physicians practice or other institution) responsible for managing the creation, transport, and storage of: Rx(s), ACP(s) and Pump history files, and an extra-institutional portion 16 located outside of that institution. In this example, the intra-institutional portion 14 comprises a server 18, which may be a computer (e.g., hosting a pump server 18a, a web server 18b, and a database server 18c or a combination of servers) or multiple computers (e.g., hosting a pump server 18a, a web server 18b, and a database server 18c or combination of servers), one or more intra-intuitional user interfaces 20 (e.g., personal computers or personal digital assistants (PDAs) 24), one or more intra-institutional infusion pumping devices 22 and, optionally, a telephonic modem 124. The intra-institutional server 18, user interfaces 20, intra-institutional pump devices 22 and optional telephonic modem 124 are connected to a wired or wireless network (e.g., a LAN or intranet). The server 18 may include or incorporate a pump server 18a, a web server 18b, and a database server 18c. Some or all portions of the server 18 need not be physically located within the institution, but rather may be located at some remote location in communication with the institution's network (e.g., the web server 18b may be located at another web hosting facility). Further, the server 18 may be incorporated into a single computer and communicate to user interfaces 20 and infusion pumping devices 22 via its own private network. Such a private network may or may not be connected to the institution's network.

In this example, the extra-institutional portion 16 of the system 10 comprises one or more extra-institutional user interfaces 24 (e.g., computers or PDAs), one or more extra-institutional infusion pumping devices 26 which may be connected via wired or wireless connection to the Internet and may then communicate with the server 18 through the institution's internet connection. Further, extra-institutional infusion pumping devices 26 may telephonically communicate to the inter-institutional portion 14 via an extra-institutional telephonic modem 12 which connects to an intra-institutional telephonic modem 124 which connects to an intra-institutional server 18.

Examples of the types of infusion pumping devices 22, 26 that may be used in this invention include, but are not limited to, those described in copending United States Utility Patent Applications Serial No. 11/212,931 entitled "Rotary Axial Peristaltic Pumps and Related Methods" filed August 26, 2005 and 11/523,794 entitled "Method and System for Controlled Infusion of Therapeutic Substances" filed September 18, 2006, the entire disclosures of which are expressly incorporated herein by reference.

In operation, the user interface devices 20, 24 act as browsers to web server 18b, of server 18, whereby users may view, monitor, set, reset, control and/or manage the operation of the pumping devices 22, 26 as described herein below.

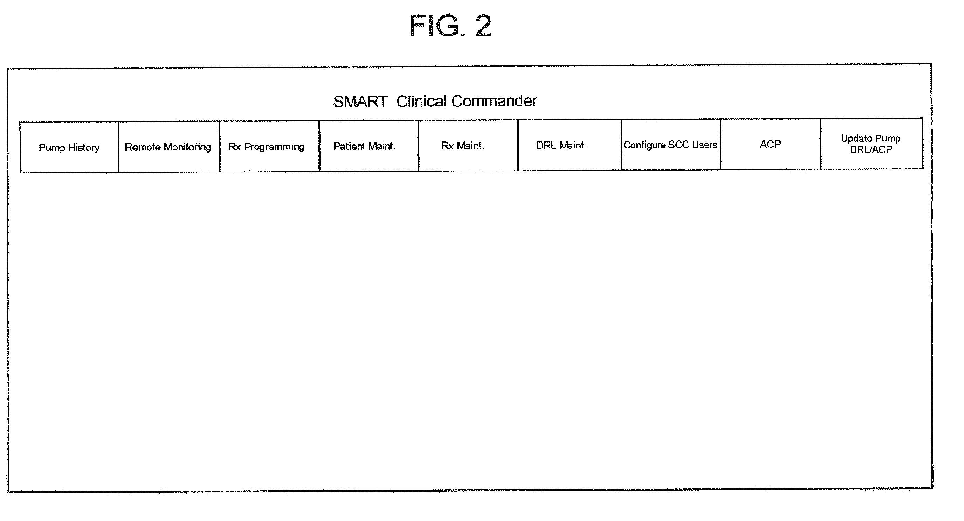

Upon accessing a login page via a web browser, the operator enters a username and password at, for example, user interface 24. After completing the login, a navigation screen appears as illustrated in Figure 2. The navigation bar displays the classification of the logged-in user as: Technician, Clinician or Administrator/Pharmacist. The enabled selections on the navigate bar are based on the user classifications as provided in the following table.

| **Function** | **Administrator Pharmacist** | **Clinician** | **Technician** | **Page Launched** |
|---|---|---|---|---|
| Pump History File | X | X | X | Pump History File |
| Remote Monitoring | X | X | | Remote Monitoring |
| Rx | X | X | | Remote Access / Programming |
| Programming Patient Maintenance | X | X | | Patient Maintenance |
| Rx Maintenance | X | X | | Rx Record Maintenance |
| Drug Reference Library Maintenance | X | | | Drug Reference Library (DRL) Maintenance |
| Administration Configuration Parameters | X | | | Administrator Configuration Parameters (ACP) Maintenance |
| Update Pump DRL/ACP | X | | | Update Pump DRL and/or ACP |

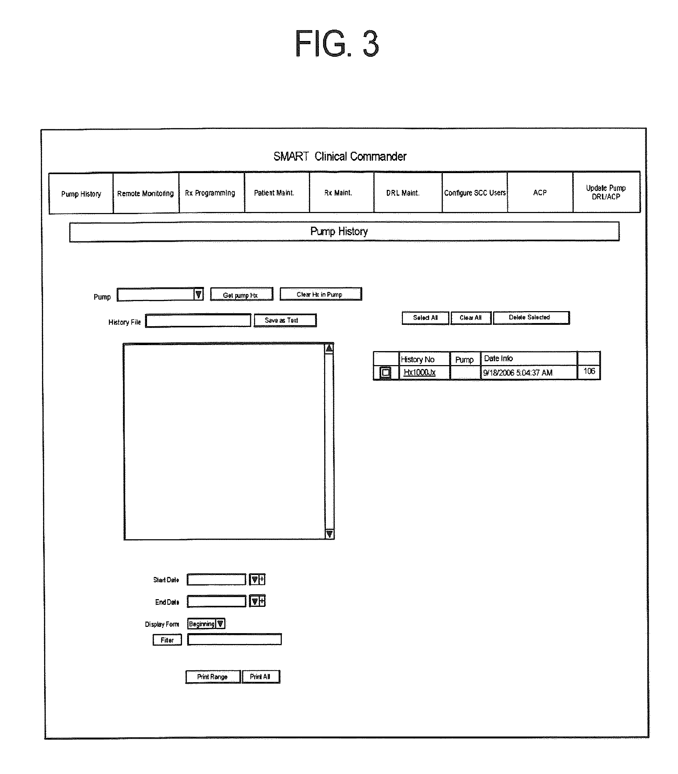

If a user selects the "Pump History File" function, a history file (Hx) directory window of saved pump history files existing in the database is provided. In addition, a scrollable Hx text window is provided to display the contents of a pump history file when the file is selected from the directory, as illustrated in Figure 3. In this example, the pump history files, as sent from the pump via the network, are comprised of at least two files- an index file and a data file. The table is sortable by clicking on any column header (e.g. history file name, date, time size), and clicking on any file name shown in the directory table will cause the contents of that history file to be displayed in the Hx text window. Files may be selected, cleared, and deleted using the window, as readily understood by the skilled artisan.

A pump pull down list allows the user to display a list of serial numbers for all pumps currently connected to the server. A "Get Pump" Hx function obtains the pump history file for the selected network-connected pump. When a network-connected pump is selected, a filename is constructed and entered in both the directory table and the "History File" text box, and the Pump Hx text window displays the contents of the history file. The displayed files may then be saved.

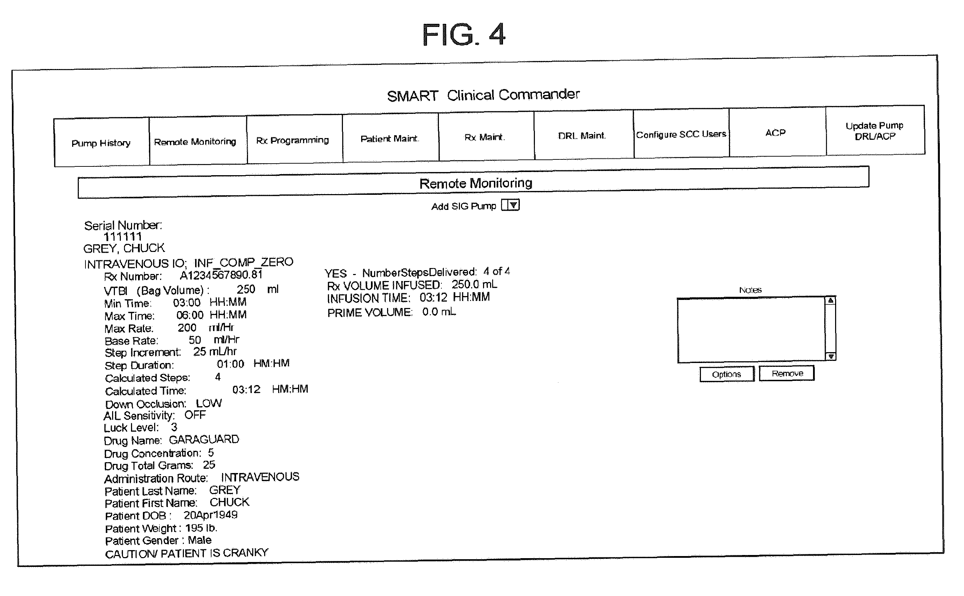

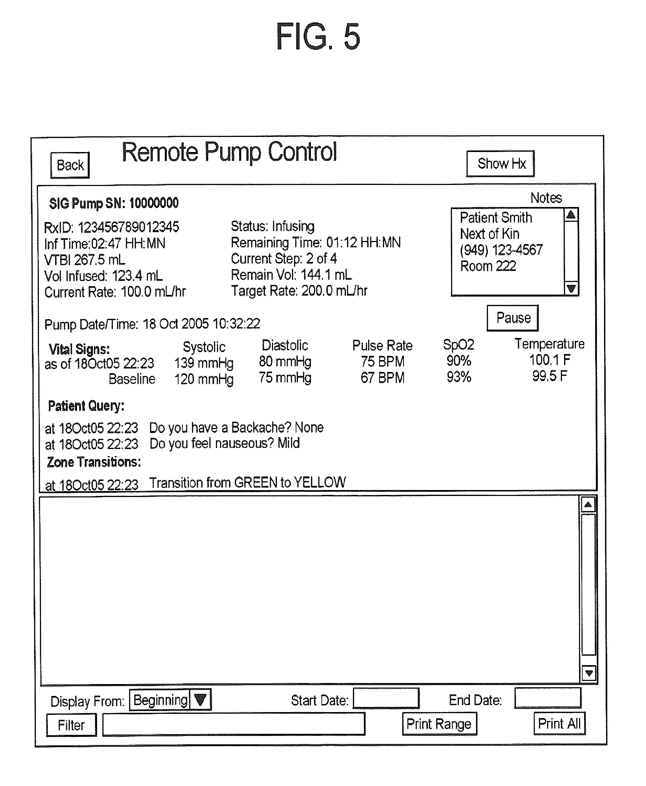

Figure 4 shows an example of a Remote Monitoring page. In this example, the remote monitoring page provides the user with the capability to monitor one or more network-connected pumps. A user may also select network-connected pumps for display, and remove displayed pumps from monitoring. Data is provided by each network-connected pump to the system for populating the remote monitoring page. The system also provides the user with the ability to present notes with respect to each connected pump, such that other users are able to read them. In order for the user to switch between pumps, a tab control, having one pump per tab, may be selected. The tab can include, for example, the pump serial number, patients name, the current status of the monitored pump, etc. Additional information that may be acquired includes current infusion status with the parameters of the currently running infusion, vital signs information with baseline information, patient query information and adverse reaction monitoring zone transition information. A "Remote Monitoring Options" page, as illustrated in Figure 5, is also available and displays the same information found on the Remote Monitoring page, with the addition of an Hx text window in which pump history file information is also displayed, and a Pause button that when activated will pause the infusion of an infusing pump being monitored.

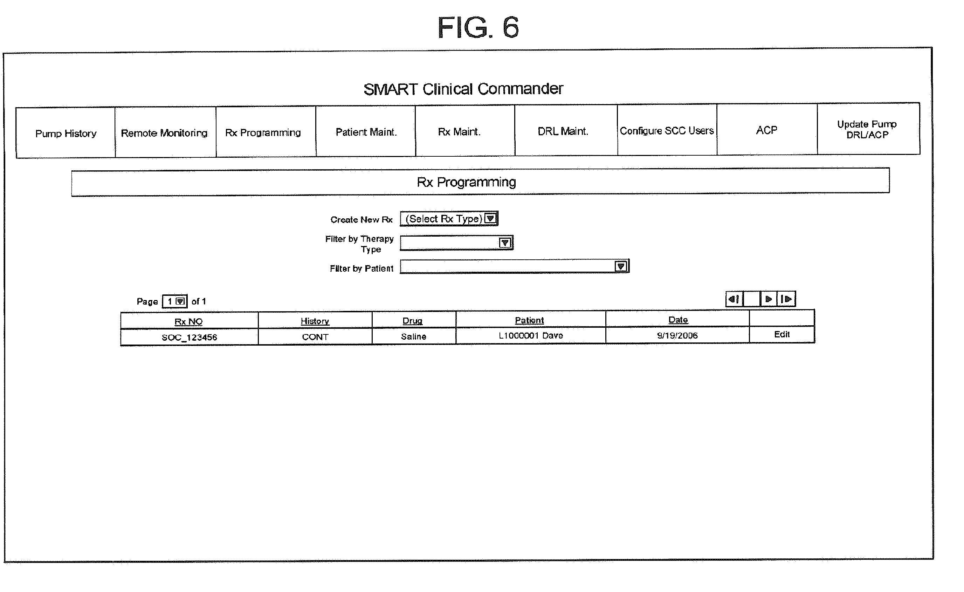

Figure 6 illustrates an "Rx Access/Programming" page which allows the operator to select an existing Rx program for editing or creating a new Rx program. An operator can open the page using a directory window of the currently existing Rx(s) in the database. At the top of the direct window are column headers (RxID, Therapy, Drug, Patient, Date, unlabeled column, etc.) that can be selected to sort the row(s) of Rx(s) according to the information in the column, as readily understood by the skilled artisan. In particular, the unlabeled column includes an "edit" control for each row, which causes the therapy specific Rx Programming page to be activated.

There are several controls and fields common to IVIG, SCIG, Continuous, Intermittent and TPN Rx Programming pages, and include, among other things, safety options, drug data and patient data fields in common, as explained below.

Safety Option Defaults: If pre-determined safety option defaults have been selected, the default values for the Rx will be set according to the selection. Default Safety options, such as Down Occlusion, AIL Sensitivity, and Lock Level, can be selected from parameters suggested from the table below:

| **Field Name** | **Field Type** |
|---|---|
| Down Occlusion | Selection: Low, High |
| AIL Sensitivity | Selection: 0.1, 0.5, 1.0, Off |
| Lock Level | Selection: 1, 2, 3 |

Programmable fields for drug data include:

| **Field Name** | **Field Type** |
|---|---|
| Drug Name | Selection: "New" and list of all Drug Reference Record (DRRs) available |

Drug Name pull down list for IVIG Rx Programming page: The name of the DRR(s) on the pull down list includes the name of the drug, the concentration, units of concentration, and route. Once a DRR has been selected, the IVIG Rx Programming page, Drug fields of concentration, total grams, route and DRR notes are populated. A "new" selection allows the user to manually fill-in the drug fields in order to define a drug not found in the DRL.

Drug Name pull down list for non-IVIG Rx Programming page: The name of the DRR(s) on the pull down list on the non-IVIG Rx Programming page include the name of the drug only. Once the drug name from DRR has been selected, the name of the drug and any associated DRR notes are displayed. A "new" selection allows the user to manually fill-in the drug name field in order to define a field not found in the DRL.

Patient Data Fields: Patient information includes patient name, date of birth, weight, weight units, gender and patient notes. On the Rx Programming pages, these fields are read. The user may select a patient from the existing database of patients using the patient name pull down list. Patient data fields are represented in the following table, although not limited to such data.

| Field Name | Field Type |
|---|---|
| Patient Name | Selection: "New" and list of all patients available |
| Patient DOB | Date |
| Patient Weight | Numeric with weight units |
| Patient Gender | Selection: M or F |

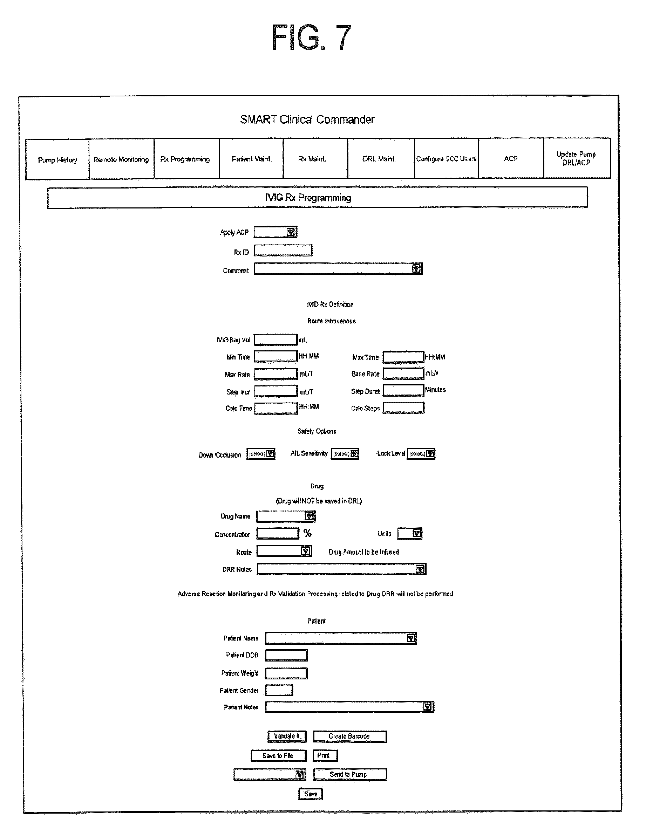

Figure 7 illustrates an exemplary "Intravenous Immune Globulin (IVIG) Rx Programming" page. This page allows data entry fields to program an IVIG Rx, as described below. Data entry fields are validated against limits when the user activates the Validate, Save, Save to files, or Save to pump buttons. The validation requires that user programmed fields be checked against factory limits and optionally against ACP limits if one was previously selected. Additionally, if a DRR has been associated with the Rx, the limits established by the DRR are also checked. Programmable fields for the IVIG Rx include Rx number, comments, route, IVIG Bag Vol., Min. Time, Max. Time, Max. Rate, Base Rate, Step Increment, Step Duration, Calculated Time, Calculated Steps and Drug Amount to be infused.

Calculated steps and time: The fields "calculated steps" and "calculated time" are not data entry fields, but rather calculated values. A DLL is provided that will compute the calculate steps and time, and are calculated after values of fields Volume TBI, Min. Time, Max. Time, Max. Rate, Base Rate, Rate Increment and Step Duration have been entered.

Drug name pull down list: The pull down list displays the DRR(s) from the DRL that are created for IVIG therapies. The entry in the pull down list shows the drug name, concentration, units and route. Upon selection of a drug name, information of the drug name, concentration, units, total grams, and route are populated. Once a valid DRR has been selected, a validate button may be used to validate the Rx against the DRR. Also, the Save, Save to File, or Save to Pump button may also validate the Rx against the DRR selected.

Drug Amount to be Infused: The amount to be infused in mg, is a calculated field, calculated by multiplying concentration (%) times IVIG Bag volume.

Drug Messages: Optional messages can be displayed in the event a user selected a "new" drug selection and manually enters drug information.

Patient name pull down list: This list displays the available patients in the patient database. Once a patient has been selected, date of birth, weight, gender and patient notes are populated in the database information. Once a valid patient record and DRR has been selected, a validate button may be used to validate the DRR and patient information.

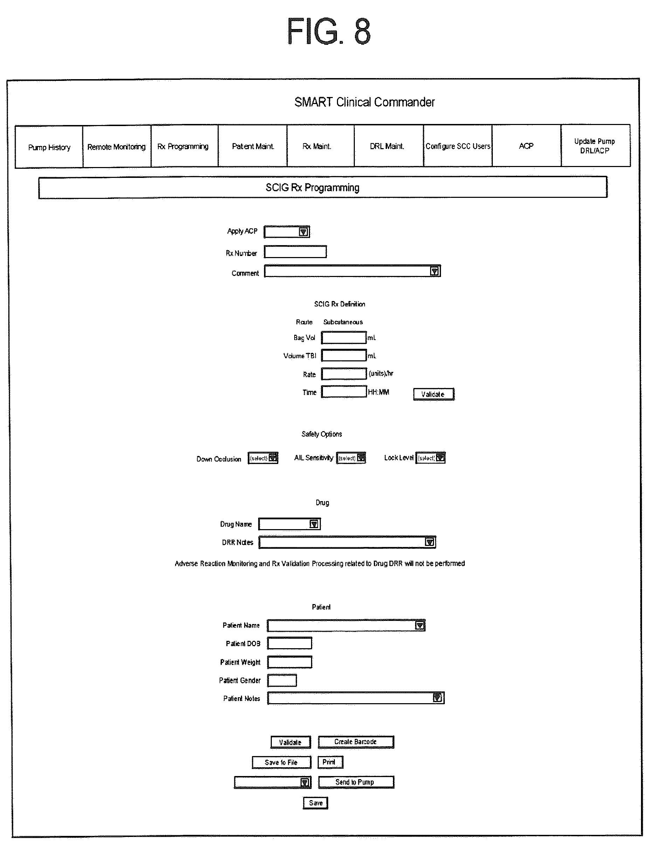

Figure 8 illustrates an exemplary "Subcutaneous Immune Globulin (SCIG) Rx Programming" page. This page provides data entry fields to program a SCIG Rx, as described below. Data entry fields are validated against limits when the user activates the Validate, Save, Save to File, or Save to Pump buttons. The validation requires that user programmed fields be checked against factory limits and optionally against ACP limits if one was previously selected. Additionally, if a DRR has been associated with the Rx, the limits established by the DRR are also checked. Programmable fields for the SCIG Rx include Apply ACP, Rx Number, Comment, Route, Bag Vol., Volume TBI, Rate and Time.

Inter-dependent Calculation Rules for SCIG Infusion: Three fields are involved in inter-dependent calculations (Volume TBI, rate and time). Once two of the three interdependent fields have been entered, the third can be dynamically calculated. A user change to any one of the fields causes a recalculation in which the lowest in priority of the non-user changed fields is recalculated. In the example, the order of priority, lowest being first, is: time and rate.

Drug name: A pull down list displays the DRR(s) from the DRL that were created for SCIG therapies.

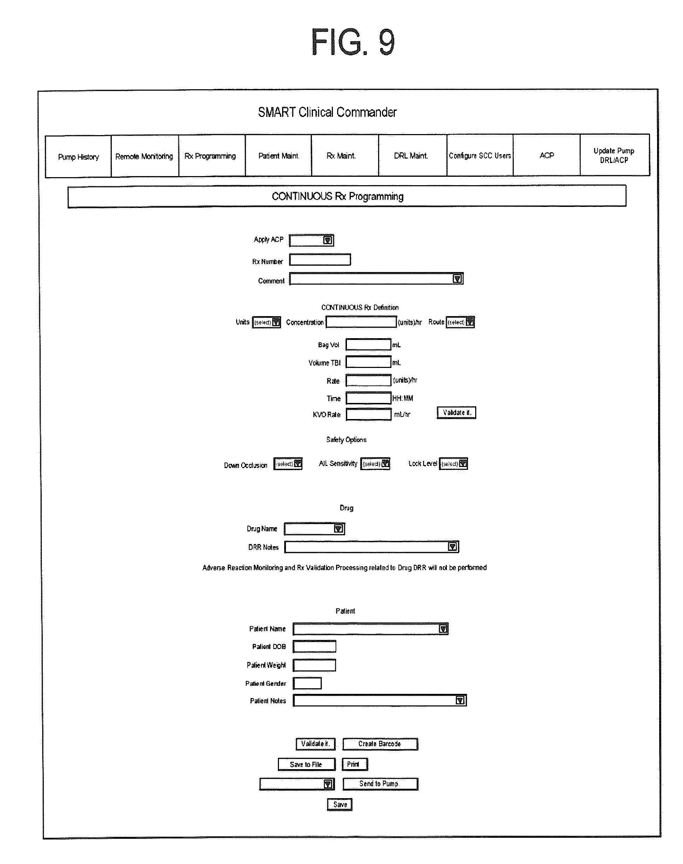

Figure 9 illustrates a "CONTINUOUS (CONT) Rx Programming" page in accordance with the invention. This page is accessible from the Rx Access/Programming page and provides data entry fields to program a CONTINUOUS Rx, as described below. Data entry fields are checked against predefined and ACP limits when the user validates the entry. The programmable fields for CONT Rx include, but are not limited to, Apply ACP, Rx Number, Comment, Units, Route, Concentration, Bag Vol., Volume TBI, Amount TBI, Rate, Time and KVO Rate.

Inter-dependent Calculation Rules for Continuous Infusion: Three fields are involved in inter-dependent calculations (Volume TBI/Amt TBI, Rate, Time). Once two of the three interdependent fields have been entered, the third can be dynamically calculated. A user change to any one of the fields causes a recalculation in which the lowest in priority of the non-user changed fields is recalculated. In the example, the order of priority, lowest being first, is: time and rate.

Concentration field: This field is disabled whenever the "units" field has been selected as mL.

Dynamic units and label: When the units field has been selected to a value other than mL (e.g., mg or mcg), the numerator of the Concentration unit and the numerator of the Rate unit changes to become the unit field selection.

Drug name: A pull down list displays the DRR(s) from the DRL that were created for CONT therapies.

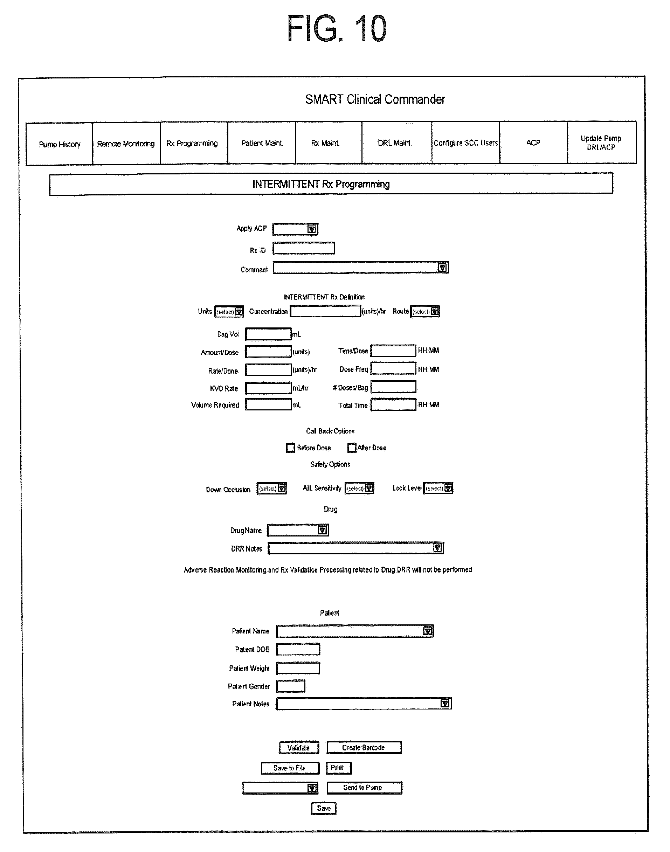

Figure 10 illustrates an exemplary "INTERMITTENT Rx Programming" page. This page provides data entry fields sufficient to program an intermittent Rx, as described herein below. Data entry fields are validated against limits when the user activates the Validate, Save, Save to files, or Save to pump buttons. The validation requires that user programmed fields be checked against factory limits and optionally against ACP limits if one was previously selected. Programmable fields include, but are not limited to, Apply ACP, Rx Number, Comment, Units, Concentration, Bag Vol., Amount and Volume of Dose, Time/Dose, Rate/Dose, Dose Freq., KVO Rate, Volume Req. and Total Time.

Call back options check boxes: When KVO rate is zero, the "Before Dose" Call Back Options check box automatically becomes checked. Otherwise, the Before Dose check box can be optionally checked by the operator.

Inter-dependant calculation rules for Intermittent Infusion: Three fields (amount/dose, time/dose and rate/dose) are related by interdependent calculation. Once two of the three fields have been entered, the third is calculated. Once all three fields have been entered or calculated, a user change to any one of the fields will cause another recalculation in which the lowest in priority of the non-user changed fields is recalculated. In this example, the order of priority, lowest being first, is: Time/Dose and Rate/Dose.

Calculated only fields: The Vol. Req. and Total Time fields are calculated fields based on the parameters: Bag. Vol., Amount/Dose, Time/Dose, Rate/Dose, Dose Freq., KVO Rate and #Dose/Bag. If parameters are changed, a these fields are recalculated.

Concentration field: This field is disabled when the "units" field is selected as mL, and concentration units is set to blank.

Dynamic units and label: Concentration units (mg/ml or mcg/ml), amount/dose (mg or mcg) or volume/dose units (mL), and rate/dose units (mg/hr, mcg/hr or mL/hr) is dynamically changed when "units" field selection is made. Additionally, the amount/dose label changes to volume/dose when the "units" selection is mL, and to amount/dose when a mg or mcg selection is made.

Drug name and message: A pull down list will display drug records created for INT therapies, and relevant messages will be displayed. For example, the message "Adverse Reaction Monitoring and Rx Validation Processing related to Drug DRR will not be performed" may be displayed as a message.

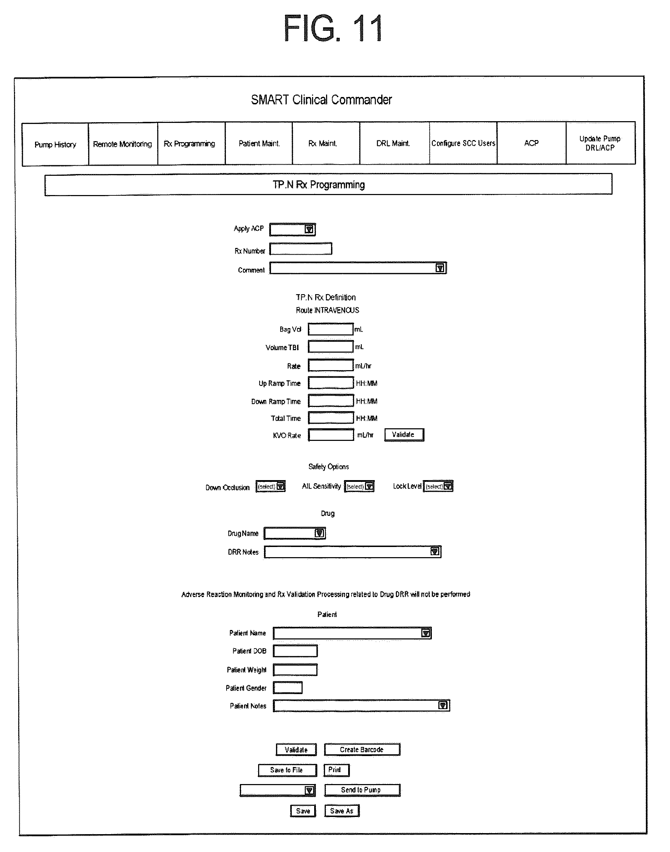

Figure 11 illustrates a "TPN Rx Programming" page. This page provides data entry fields to program a TPN Rx, as described below. Validation requires that user-programmed fields be checked against predetermined Factory and ACP limits. The programmable fields for the TPN Rx include, but are not limited to, Apply ACP, Rx Number, Comment, Units, Route, Bag. Vol., Volume TBI, Rate, Up Ramp Time, Down Ramp Time, Total Time, and KVO Rate.

Inter-dependent Calculation Rules for Continuous Infusion: Five fields are involved in inter-dependent calculations (Volume TBI, Rate, Up Ramp Time, Down Ramp Time and Total Time). Once four of the five fields have been entered, the fifth field is dynamically calculated. A change to any of the fields results in another recalculation of the lowest in priority of the remaining fields. In this example, the order or priority, lowest being first, is: Total Time and Rate.

Drug Name: A pull down list of drug records is displayed for TPN therapies.

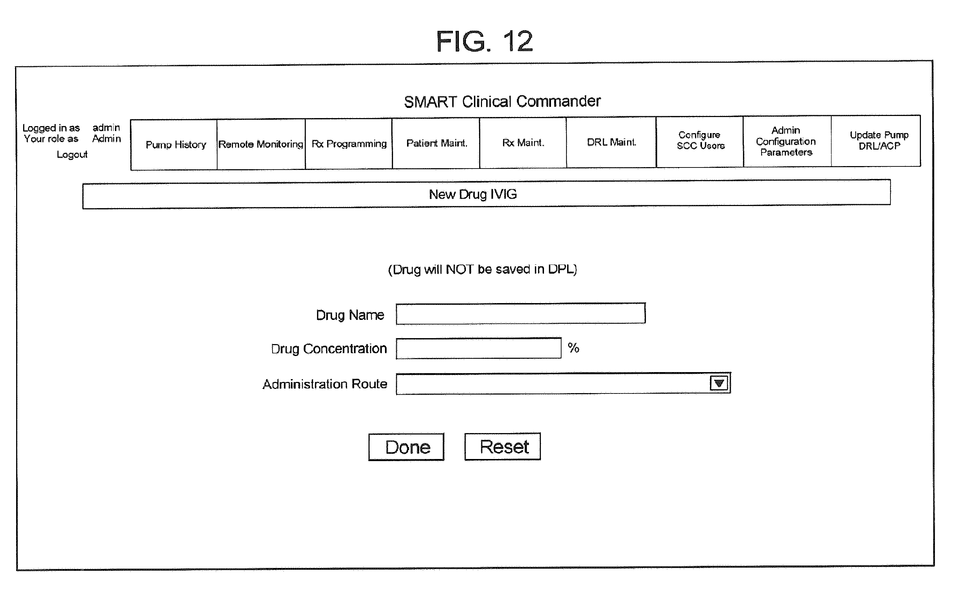

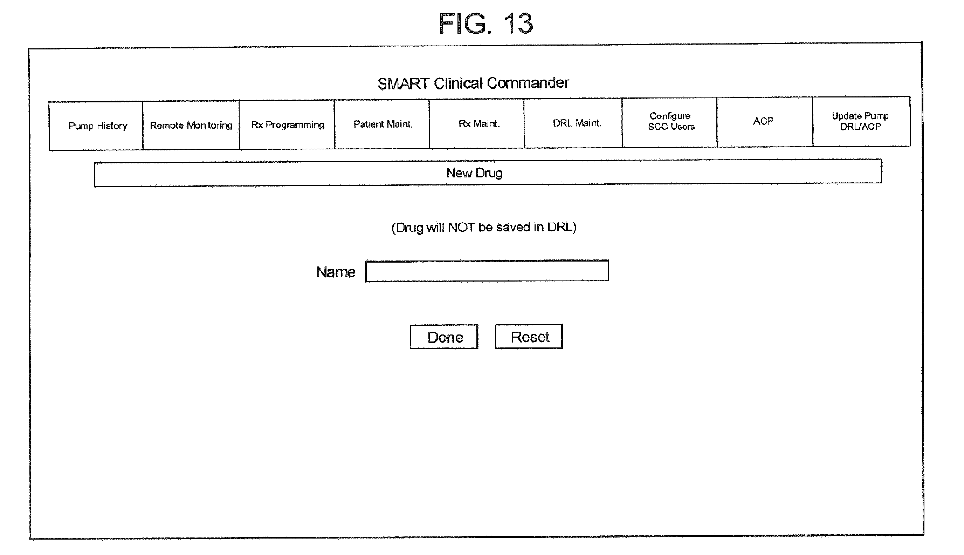

Figures 12 and 13 illustrate new "IVIG Drug" and "SCIG/CONT/INT/TPN Drug" pages. The IVIG drug page allows users to define a drug to be used with an IVIG Rx being programmed, and the SCIG/CONT/INT/TPN drug page allows users to define a drug to be used with the SCIG, CONT, INT or TPN Rx being programmed. Drugs that are created are not saved to the system, and limits are not set for these created drugs. Users may however define the drug name and concentration, for example.

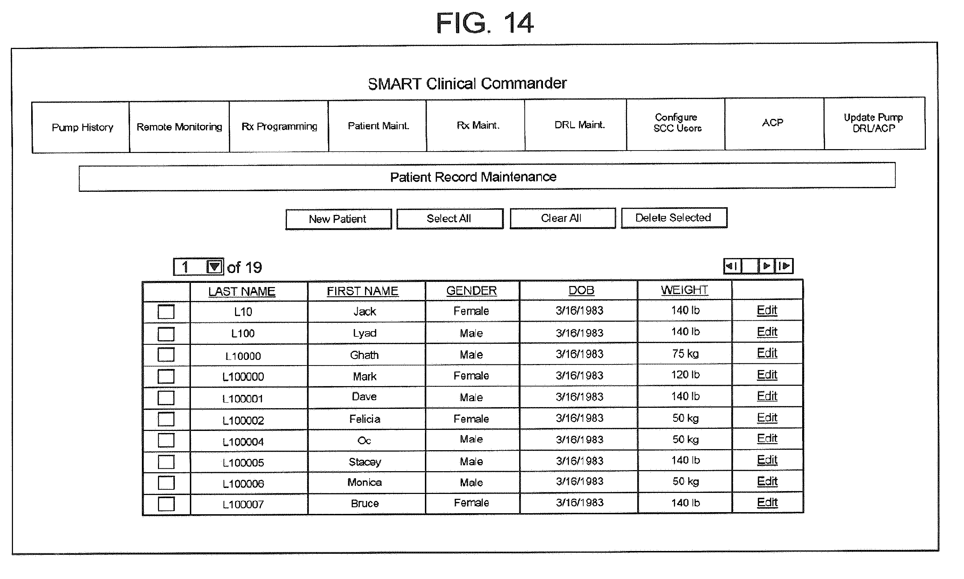

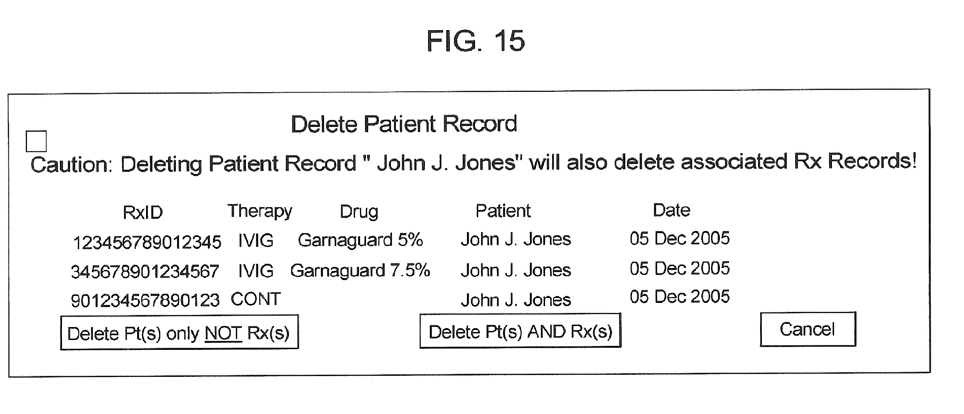

Figure 14 illustrates a "Patient Record Maintenance" page. This page allows users to create, edit and delete patient records. A patient directory displays a window of available patients. The directory displays, for example, information about the patient such as last name, first name, gender, date of birth (DOB) and weight. Figure 15 shows an exemplary illustration of a patient delete page, which displays all of the Rx(s) associated with the selected patient(s) for deletion.

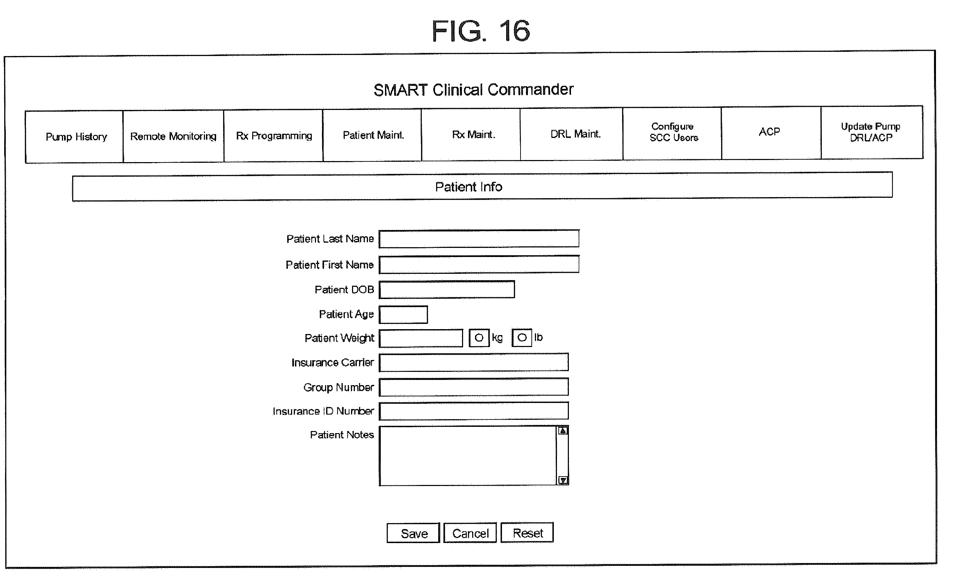

Figure 16 shows an exemplary "New Patient" page, which is accessible from the patient record maintenance screen. The new patient page allows users to input a new or modify an existing patient record, including parameters of, but not limited to: last name, first name, DOB, weight, units (of weight- lbs or kg), gender, insurance carrier, group number, insurance ID number and patient notes. When a new page is being created, the fields are initially blank. For existing patients, on the other hand, the fields are populated with information retrieved from a database for the patient. Patient information is saved by selecting the "save" button on the screen. Use of this selection causes the contents of the new patient to be saved and displayed in the patient record maintenance page. If the patient name already exists, a notification is displayed.

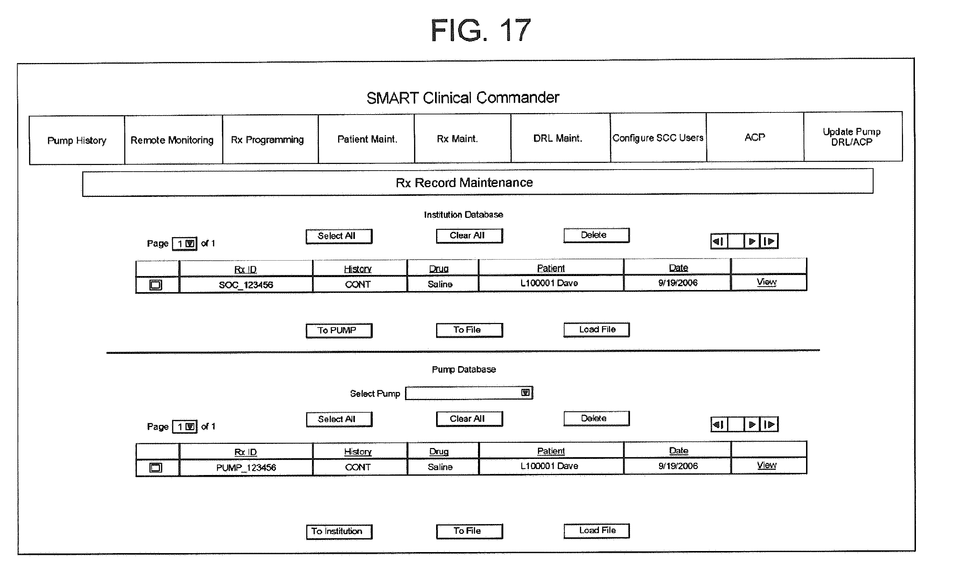

Figure 17 shows an exemplary "Rx Record Maintenance" page, which allows the user to merge an institution's Rx database with a specific pump's Rx database. Rx(s) stored in either database may also be deleted, viewed or copied to a separate folder or file. Upon accessing the Rx record management page, a "select pump" pull down lists enables the user to select a pump, while the institution database shows all Rx(s) available in the database.

Institution database directory window: The directory window displays the Rx(s) of the institution database in a scrollable and sortable directory window. Columns in the directory provide, for example, RxID, Therapy, Drug, Patient and Date.

Pump Rx database directory window: The pump database directory window displays all Rx(s) in the database of the selected pump in a scrollable, sortable window. Columns in the pump Rx database directory window include, for example, RxID, Therapy, Drug, Patient and Date.

Institution and Pump view: Selection of this button displays the contents of the selected Rx in the therapy specific Rx Programming page. No modification can be made to the Rx in this page.

Institution "to Pump", "to File" and "Load File": Selected Rx(s) can be copied from the institution database to the pump database. Conflicts may occur if the RxID number of an institution Rx is the same as an existing pump Rx. When a conflict appears, a message is displayed allowing the user to review the pump and institution Rx in order to decide which Rx should prevail. Once Rx(s) have been selected, they may be saved to a file or folder for later use (i.e. to be loaded at a later time).

Pump select: A pull down list displays the pumps connected to the host server. Selection of a pump causes the Rx(s) in the selected pump database to be downloaded to the host server and displayed in the Pump Rx Database Directory window.

Pump "to INSTITUTE", "to FILE" and "Load FILE": These buttons appear on the screen, and allow users to copy selected Rx(s) of the selected pump database to the institution database. If conflicts occur, they are resolved in the same manner as those described in Institution "to Pump", "to File", and "Load File". Once copied, the files may be saved and loaded at a later time.

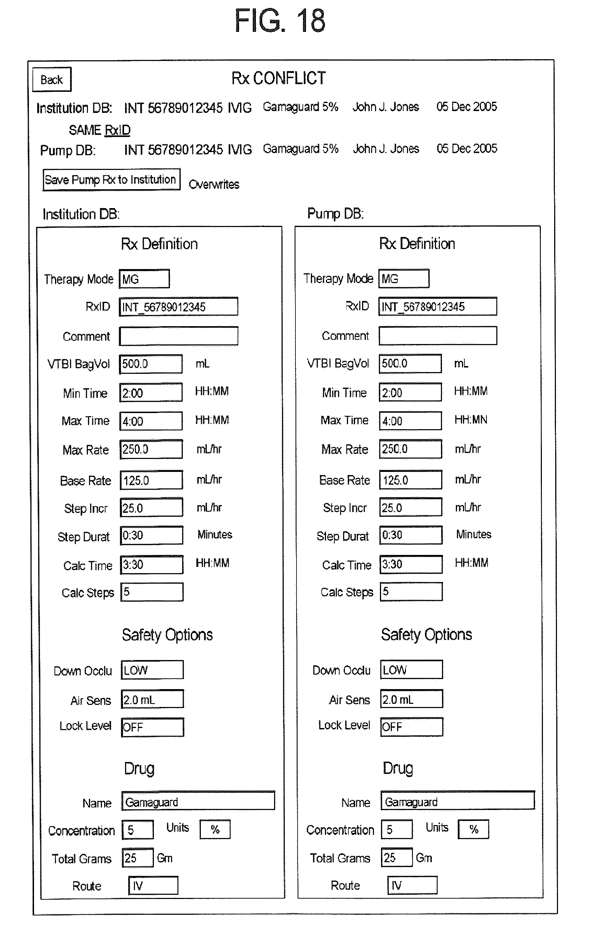

Figure 18 is an exemplary diagram of an "Rx Save Conflict" page. This page is displayed when the Rx maintenance page "to Pump" or "to Institute" are selected and the source Rx has the same RxID as an existing Rx in the destination database. Additionally, the page provides information for a user to decide which of two Rx(s), that contain the same RxID, should be saved. The page displays complete identifying information for each Rx and a side-by-side comparison to two Rx(s) so the operator can examine the contents of each Rx. Notifications of why a conflict exists may also be displayed.

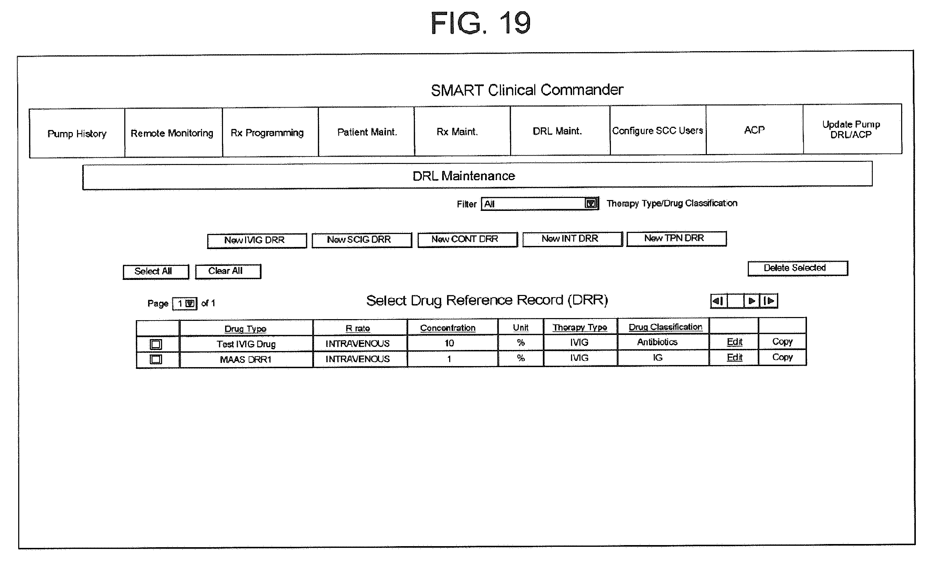

Figure 19 shows an exemplary "Drug Reference Library (DRL) Maintenance" page, which displays a directory of drug reference records (DRRs). DRR(s) are identified by drug name, route, concentration, units, and applicable therapies. Therapy types and classifications may be selected via a pull down list for all, IVIG, SCIG, CONTINUOUS, INTERMITTENT, IG, Antibiotics, Cardiovascular, TPN, Analgesics, Chemotherapy, Biologic and other types of therapies/classifications.

General provisions for DRR pages: DRR(s) are identified by a qualified DRR name. A defined DRR name includes at least a drug name, concentration, units and route. For IVIG, CONT, INT and TPN, the route is set to INTRAVENOUS and displayed on the DRR page. For SCIG, the route is set to SUBCUTANEOUS and displayed on the DRR page. Units for IVIG and SCIG DRR pages are set to "%", and units for CONTINUOUS and INTERMITTENT are selected from mL, mcg and mg. Units for TPN are set to mL.

For new DRRs, the fields are initially blank, as follows:

| **Field Name** | **Field Type** |
|---|---|
| Drug Name | Alpha-numeric |
| Concentration | Numeric |
| Units | Selection |
| Route | Read only text |

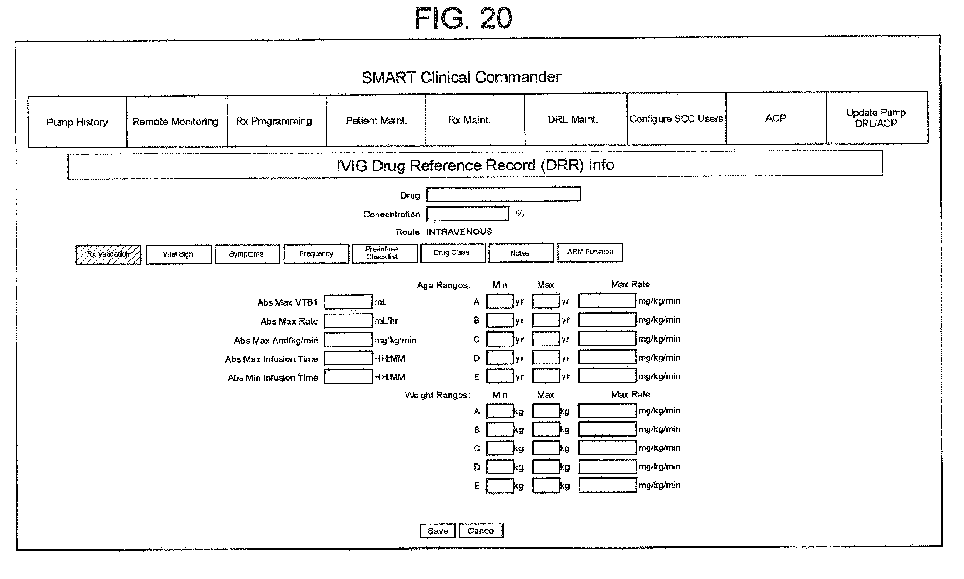

DRR page format (e.g., Fig 20): DRR pages include tabbed windows (Rx Validation, Vital Signs, Symptoms, Frequency, Pre-infusion Check List, Drug Class, Notes and ARM Function) in which the user programs specific parameters. At the top of the DRR page, the drug identifying information (Drug Name, Concentration, Units and Route) is displayed.

Figure 20 illustrates an "IVIG DRR page / Rx Validation" tab, which is accessible from the DRL maintenance page. Programmable fields include, for example, Abs Max. VTBI, Abs Max. Rate, Abs Max. Amt/kg/min., Abs Max. Infusion Time, Abs Min. Infusion Time, Age Ranges (5) A-E: Min., Age Ranges (5) A-E: Max., Age Ranges (5) A-E: Max. Rate, Weight Ranges (5) A-E: Min., Weight Ranges (5) A-E: Max. and Weight Ranges (5) A-E: Max. Rate.

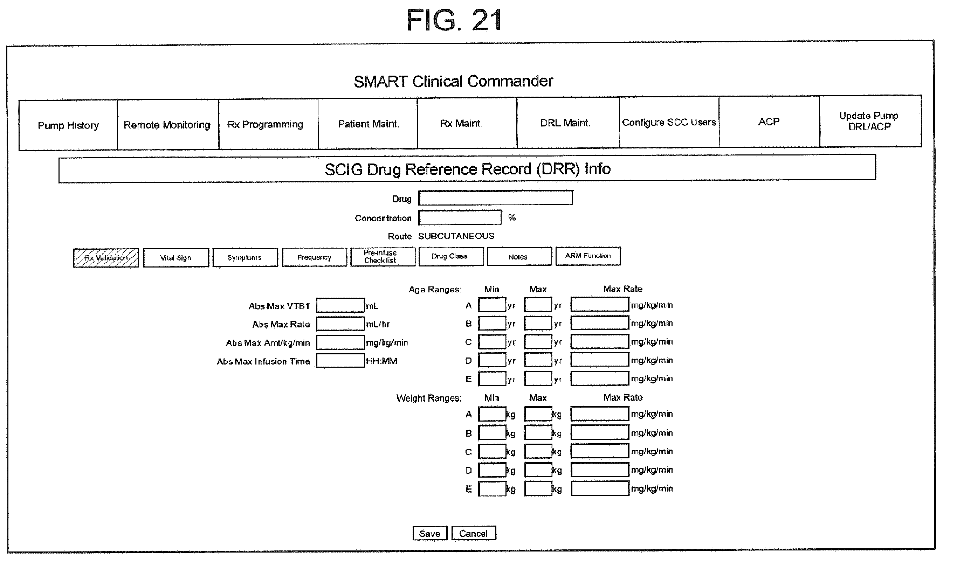

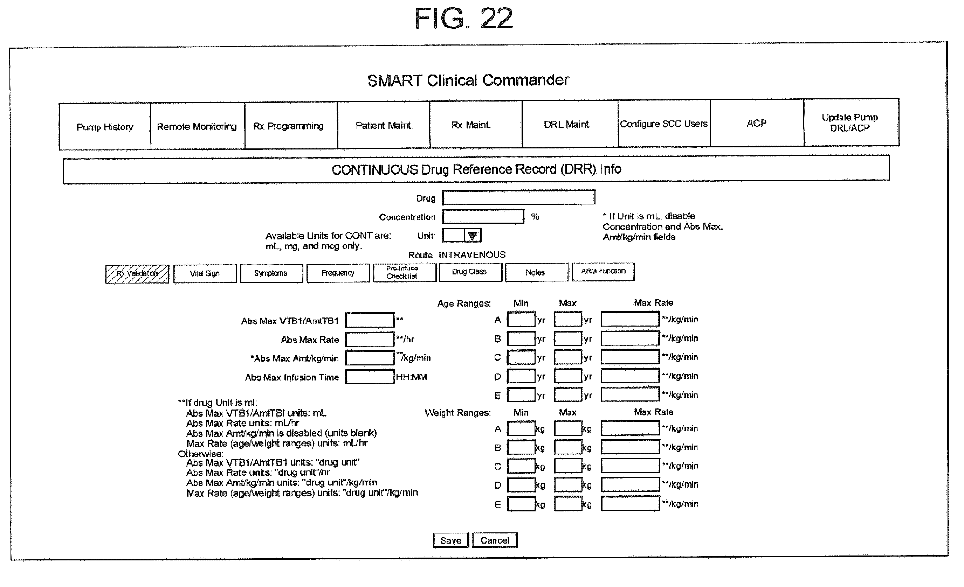

Figures 21 and 22 illustrate a "SCIG DRR page / Rx Validation" tab and a "CONTINUOUS Drug Reference Record (DRR) page / Rx Validation" tab, respectively, which are accessible from the DRL maintenance page. Programmable fields include the same as those in the IVIG DRR page, without the Abs Min. Infusion Time.

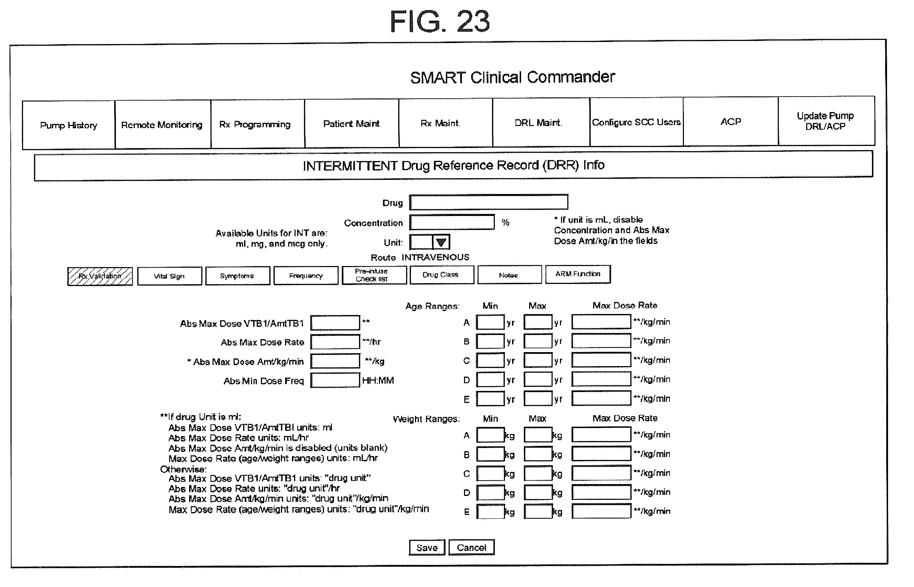

Figure 23 shows a new "INTERMITTENT DRR page / Rx Validation" tab. Programmable fields for the INTERMITTENT DRR Rx validation tab include, for example, Abs Max. Dose VTBI/AmtTBI, Abs Max. Dose Rate, Abs Max. Dose Amt/kg/min, Abs Min. Dose, Freq., Age Ranges (5) A-E: Min., Age Ranges (5) A-E: Max., Age Ranges (5) A-E: Max. Dose Rate, Weight Ranges (5) A-E: Min., Weight Ranges (5) A-E: Max., Weight Ranges (5) A-E: Max. Dose Rate. Programmable fields are similar for TPN DRRs as well, without dosage levels.

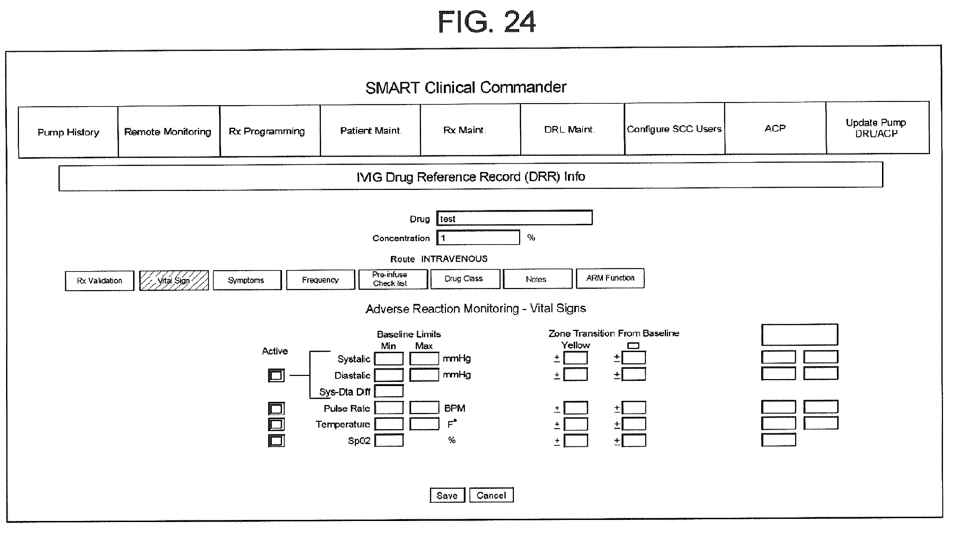

Figure 24 shows a "DRR Vital Signs" tab, accessible from the DRR page. The DRR Vital signs tab allows the user to design the vital sign absolute limits, vital signs baseline limits, and yellow and red zone transition thresholds. Available vital signs include: Systolic / Diastolic blood pressure, the difference between Systolic and Diastolic measurements, Pulse Rate, Temperature and SpO2. There are multiple programmable fields that include, but are not limited to, baselines for systolic, diastolic pressure, pulse, temperature, SpO2, etc.

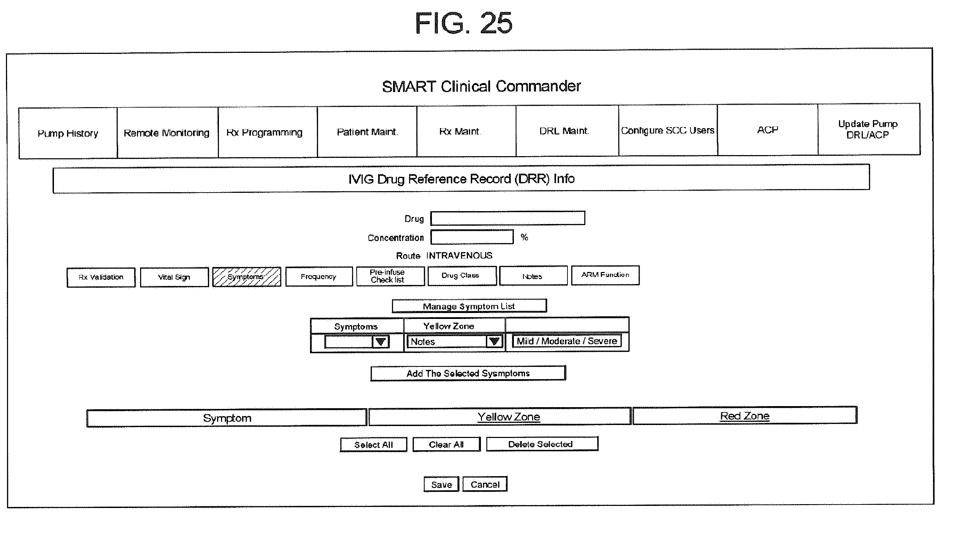

Figure 25 illustrates an exemplary "DRR Symptoms" tab, which is accessible from the DRR page. The symptoms tab allows the user to enable queries about a patient's symptoms, and to configure the queries. Upon selection of the symptoms tab, the symptoms window can display pre-configured symptoms or a single row of an un-configured symptom query. Options include:

Manage Symptom List: Displays the DRR Add/Delete Symptoms page.

Symptom Pull Down List: The user may select a symptom from those provided form the symptom Pull Down List.

Yellow Zone Pull Down List: The user may select a yellow zone response from those provided from the Yellow Zone Pull Down List. Once the Yellow Zone response is selected a resultant Red Zone response is automatically made according to the following table.

| **Yellow Zone pull down list selection** | **Resultant Red Zone setting** |
|---|---|
| None | Mild/Moderate/Severe |
| Mile | Moderate/Severe |
| Moderate | Severe |
| Severe | None |
| Mild/Moderate | Severe |
| Mid/Moderate/Severe | None |

Add the Selected Symptom: Add the Selected Symptom creates a new box, the symptom name, yellow zone response criteria, and red zone response criteria.

Symptom check box: The user can select the symptom for deleing.

Delete Symptom: Allows a user to delete the selected symptom row.

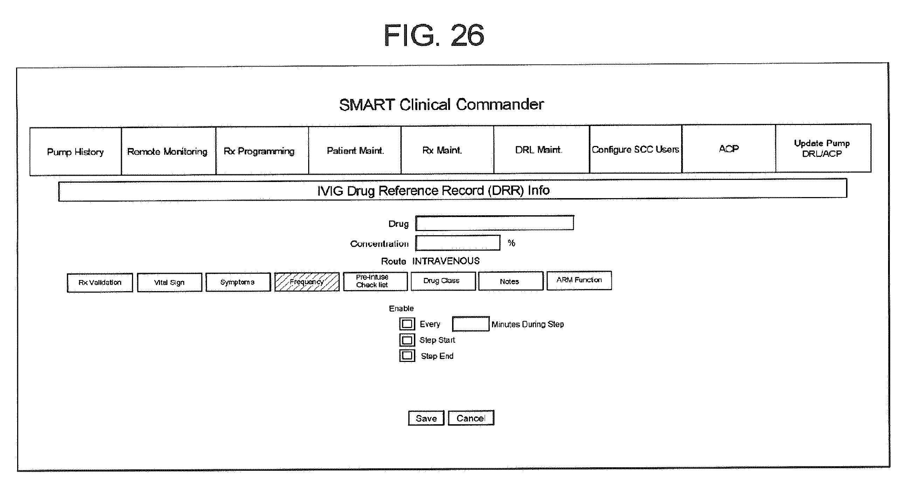

Figure 26 illustrates an exemplary "DRR Frequency" tab, accessible from the DRR page. The Frequency tab allows the user to select when to acquire Vital Sign and Symptom Query information. At least one of three choices are selected in order to enable Vital Sign/ Symptom acquisition: one, some or all.

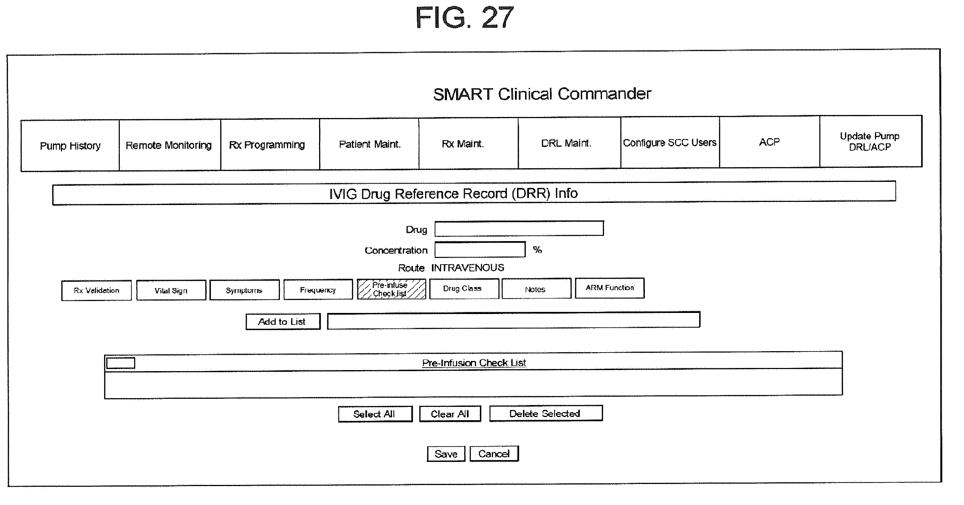

Figure 27 shows a "DRR Pre-Infusion Check List" tab, which is also accessible from the DRR page. The Pre-Infusion Check List tab allows the user to design additional pre-infusion check items to be displayed to the pump operator when an infusion is started. The pump operator acknowledges each pre-infusion check list item before the infusion begins. The operator can enter the desired text that will appear in the Pre-Infusion Check list on the pump when infusion begins, as well as delete checked pre-infusion check list items upon completion of task.

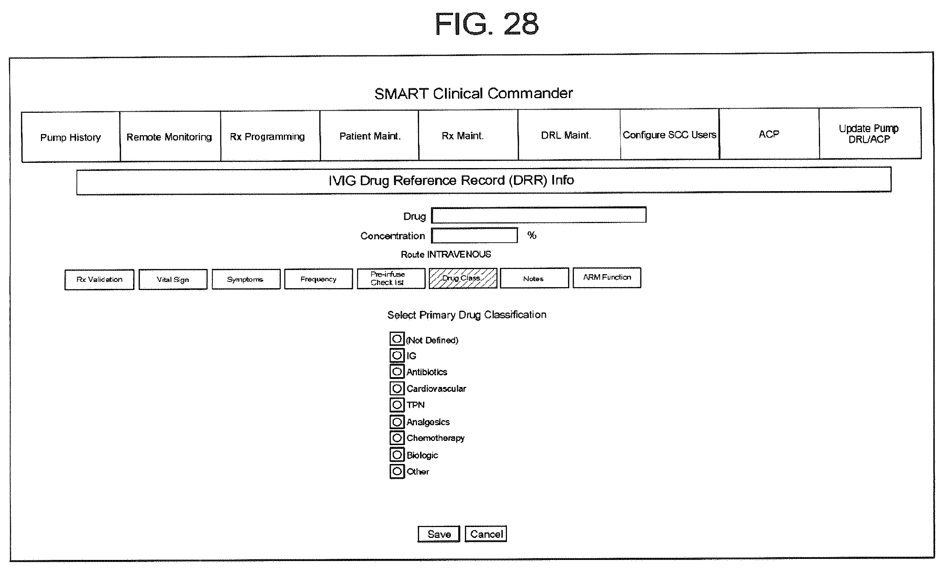

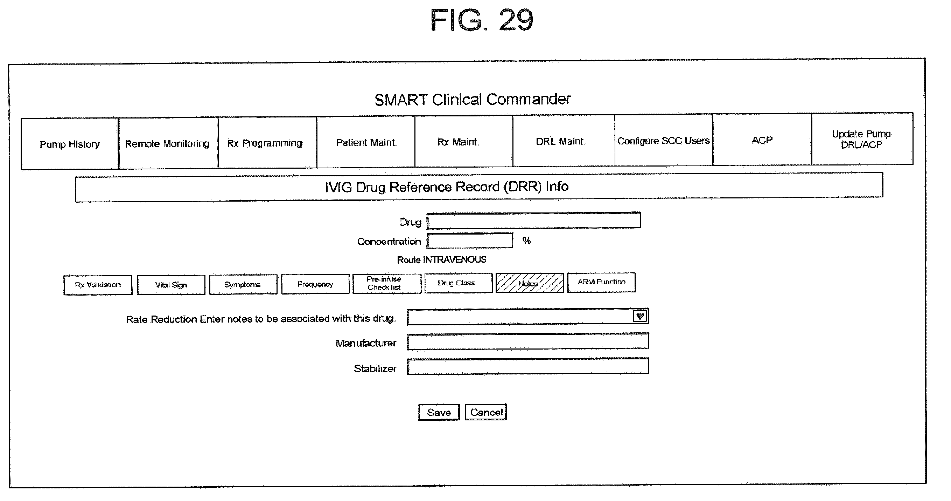

Figure 28 shows a "DRR Applicable" tab page, accessible from the DRR page. The Applicable tab page allows the user to select which drug classification (IG, Antibiotics, Cardiovascular, TPN, Analgesics, Chemotherapy, and other) the DRR is applicable to. Figure 29 shows a "DRR Notes" tab, which allows the user to provide text notes, the name of the manufacturer of the drug and the stabilizer used in the drug.

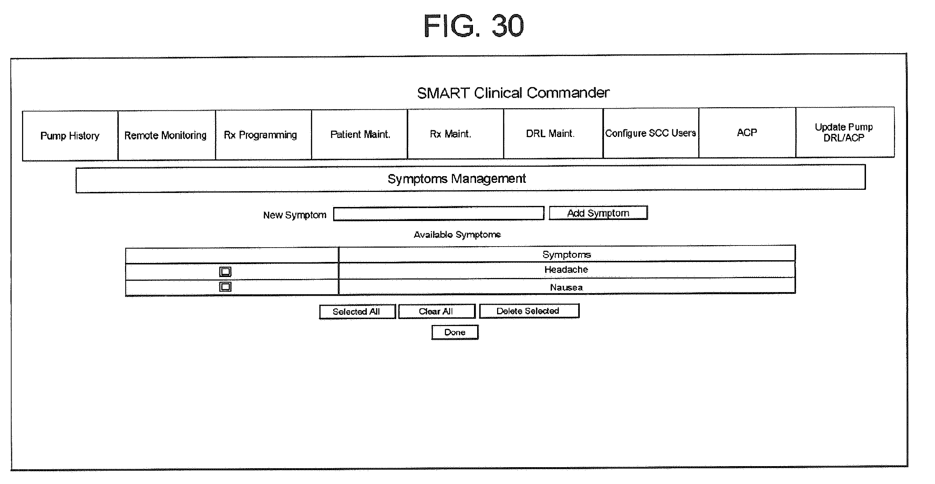

Figure 30 shows a "DRR Add/Delete Symptoms" page, accessible from the DRR page/Symptoms tab. The Add/Delete page allows the user to add new symptoms which can be used for symptom selection on the DRR/Symptoms tab, Symptoms pull down list. The page shows an available symptom list, a new symptom text box and multiple buttons.

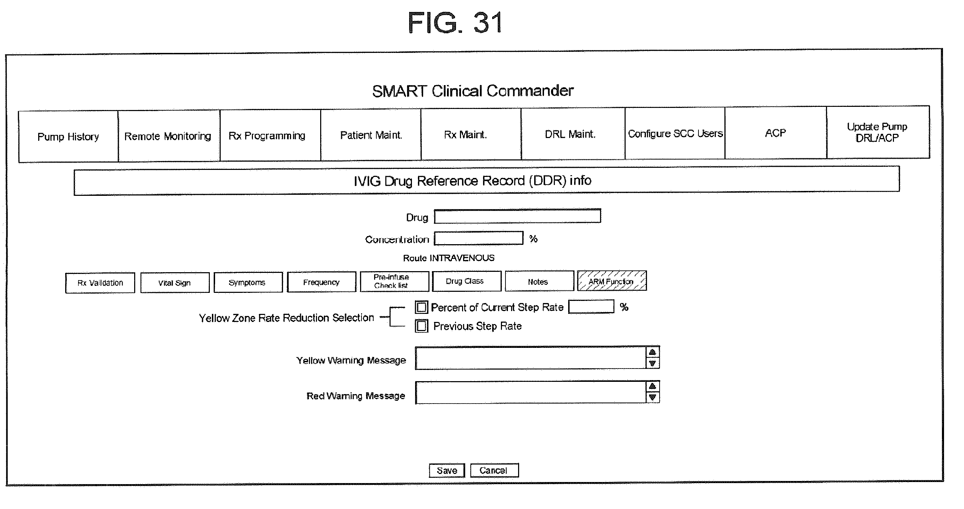

Figure 31 is an exemplary "DRR ARM Function" tab, also accessible from the DRR page. This page allows a user to select a various zone rate selections and display warning messages for each zone.

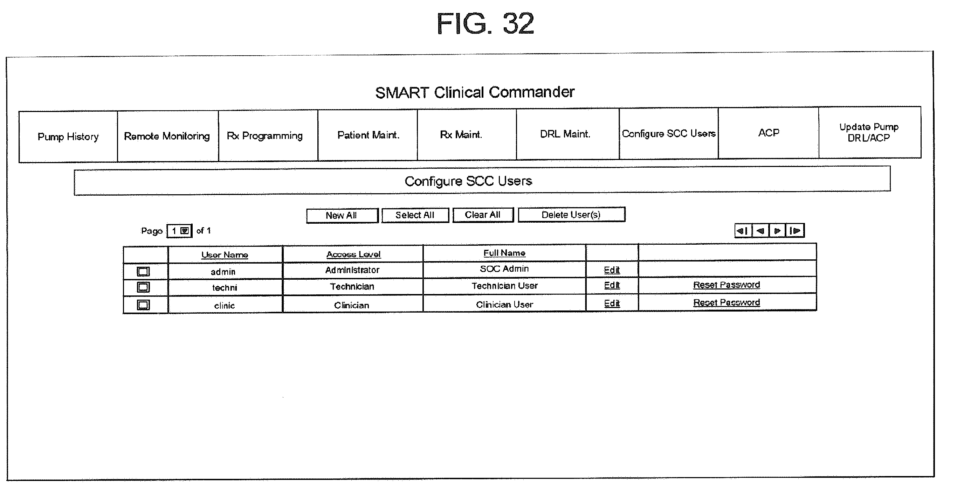

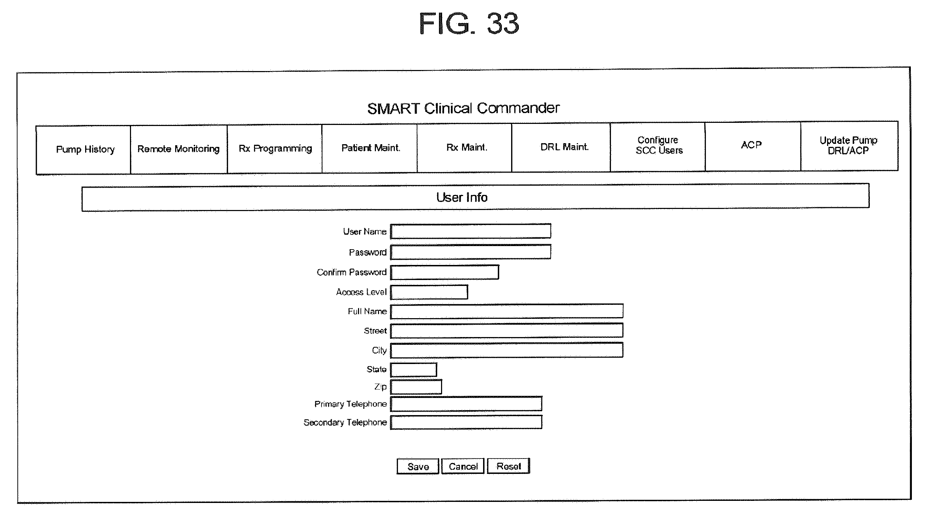

Figure 32 shows a "Configure SCC Users" page, accessible from the navigation bar, which allows users to create, edit and delete authorized users of the system. The "Add/Edit User" page (Figure 33), accessible from the Configure SCC Users page, allows users to add users and edit user fields, including username, password access level, etc.

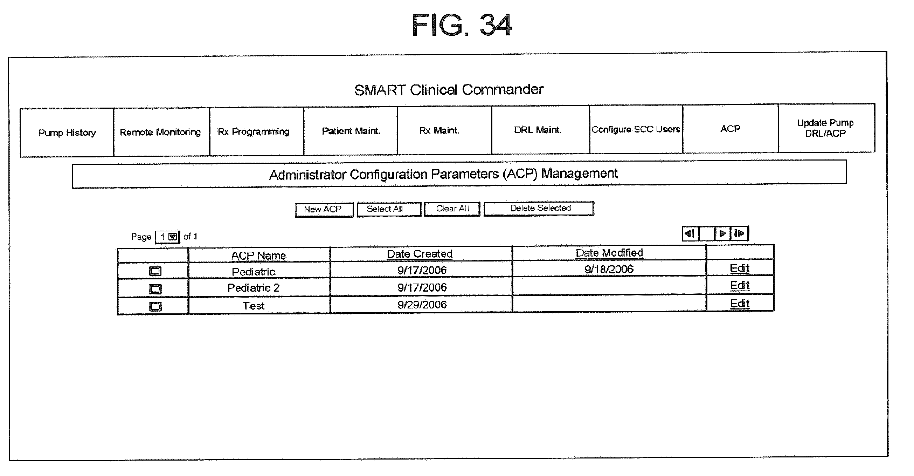

Figure 34 illustrates an "Administrator Configuration Parameters (ACP) Maintenance" page, accessible from the navigation bar. This page allows the user to select an ACP for editing, deleting one or more ACP(s), and creating a new ACP. The ACP page also includes a tabbed window in which the user can program parameters.

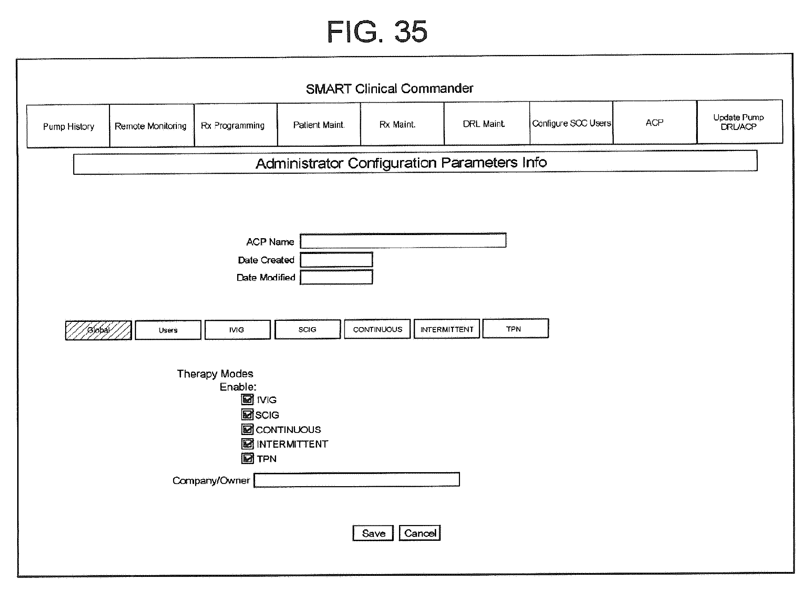

ACP Global tab (Figure 35): Allows users to enable and disable the therapeutic modes of the pump. For each therapeutic mode, a check box is provided which must be checked to enable the various therapeutic modes.

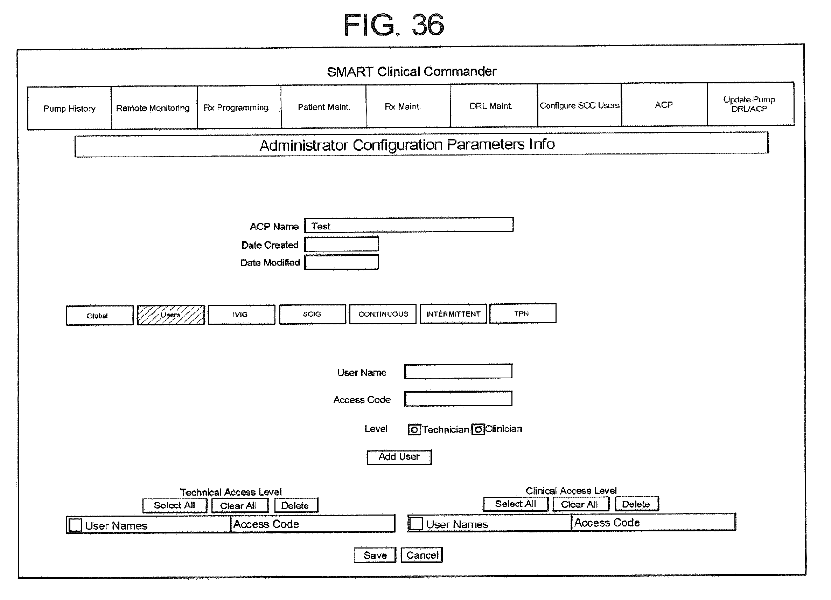

ACP Users tab (Figure 36): Allows users to configure technician and clinician level access for the pump. For each technician and clinician, a username and access code is required.

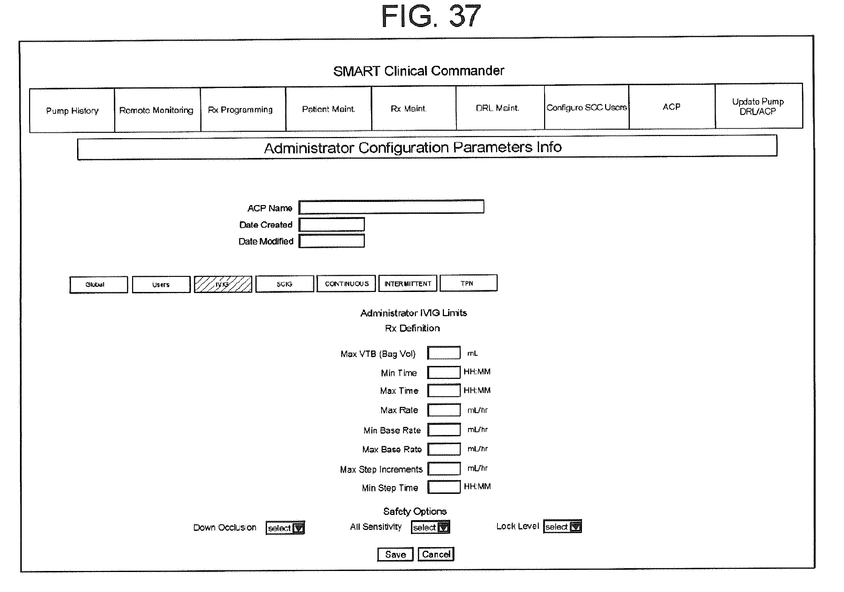

ACP IVIG tab (Figure 37): Allows users to configure the IVIG Rx parameter Min/Max limits and default safety options.

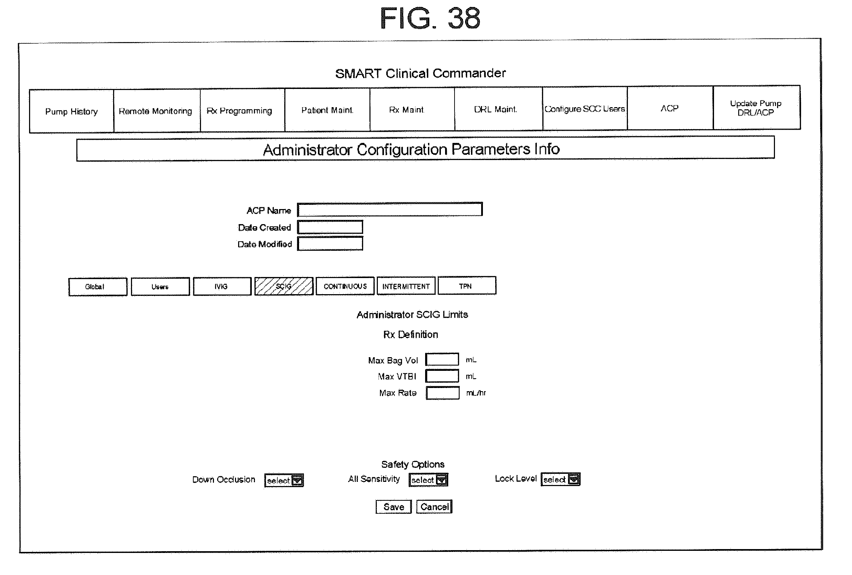

ACP SCIG tab (Figure_38): Allows users to configure the SCIG Rx parameter Min/Max limits and default safety options.

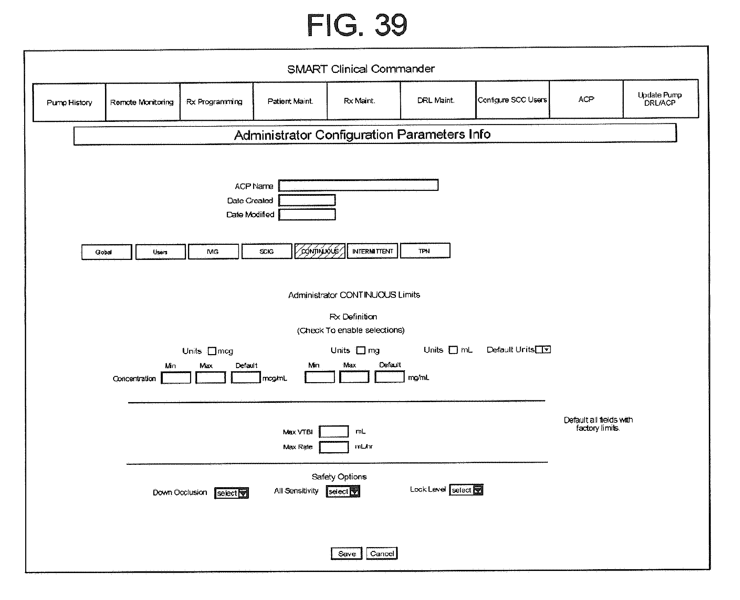

ACP CONT tab (Figure 39): Allows users to configure the CONT Rx parameter Min/Max limits and default safety options.

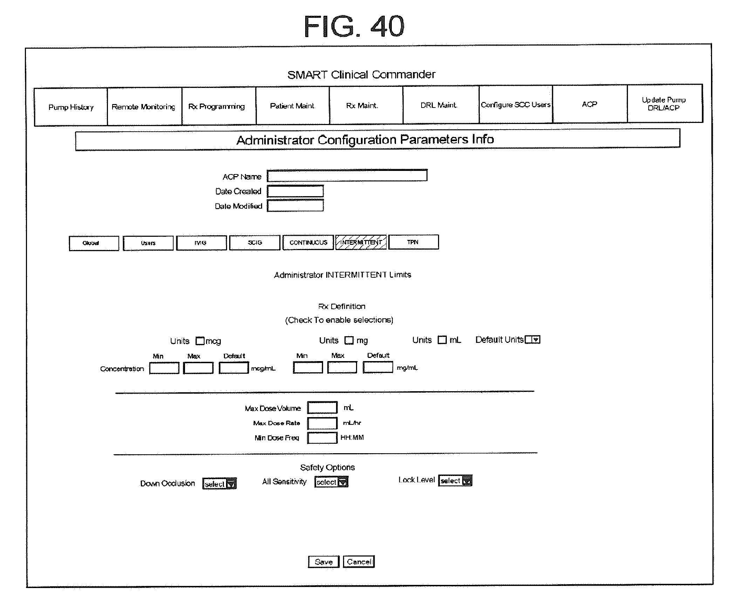

AC INT tab (Figure 40): Allows users to configure the INT Rx parameter limits and default safety options.

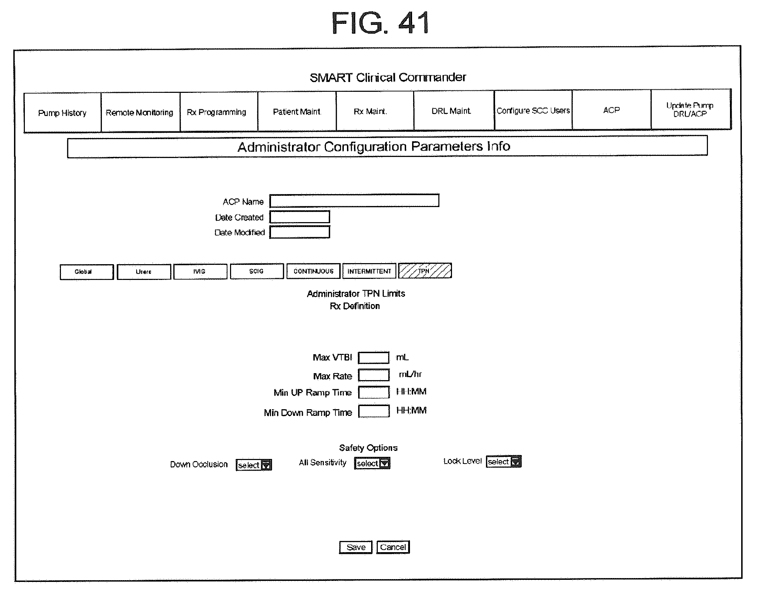

ACP TPN tab (Figure 41): Allows users to configure the TPN Rx parameter limits and default safety options.

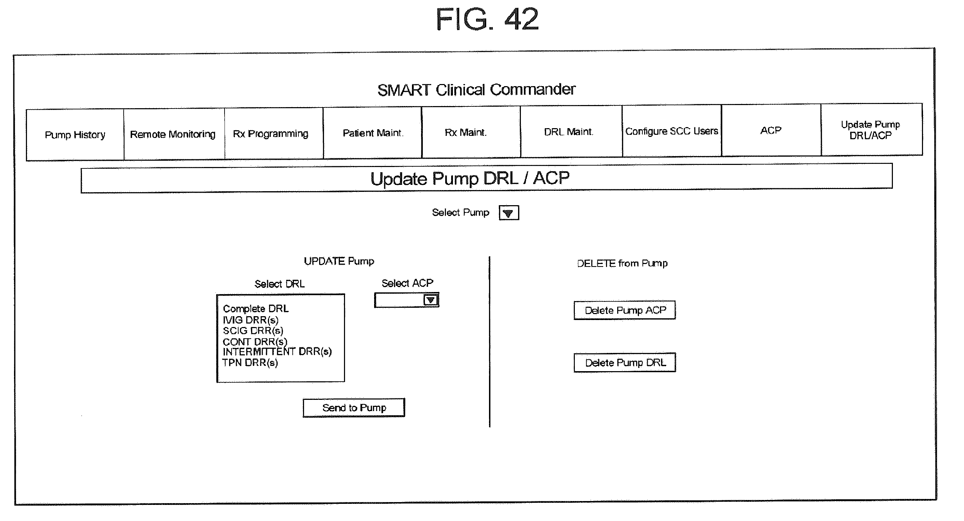

Figure 42 shows an "Update Pump DRL and/or ACP" page, that is accessible from the navigation bar page. This page allows users to copy the DRL and/or a selected ACP to a server-connected pump. The page displays, for example, the following controls: Select Pump, Select DRL Pick List, Select ACP pull-down selection, Send to Pump button, Delete Pump ACP button and Delete Pump DRL button.

Those of skill in the art will appreciate that the system and method of the present invention may be used for many types of infusions. One particular use is in the administration of Immune Globulin (Ig) therapy. Immune Globulin may be infused intravenously (e.g., Intravenous Immune Globulin (IVIG) Therapy) or subcutaneously (e.g. Subcutaneous Immune Globulin (SQIG) therapy). Immune Globulin therapies have been used to treat primary immunodeficiencies (e.g., congenital agammaglobulinemia, hypogammaglobulinemia, common variable immunodeficiency, X-linked immunodeficiency with hyperimmunoglobulin M, severe combined immunodeficiency (SCID) and Wiskott-Aldrich syndrome). Also, IVIG therapy may be used in the treatment of Kawasaki Syndrome, B-Cell Chronic Lymphocytic Leukemia, Idiopathic Thrombocytopenic purpura (ITP), acute graft-versus-host disease associated interstitial pneumonia (infectious or idiopathic) after bone marrow transplantation (BMT), human immunodeficiency virus (HIV), as a treatment for Acute Guillain-Barra Syndrome, refractory dermatomyositis, hperimmunoglobulinemia E syndrome, Lambert-Eaton Myasthenic Syndrome, Relapsing-Remitting Multiple Sclerosis, Parvovirus B19 Infection and associated anemia, Chronic Inflammatory Demyelinating Polyneuropathies, Multifocal Motor Neuropathy (MMN), infectious diseases, adrenoleukodystrophy, acquired Factor VII inhibitors, acute lymphoblastic leukemia, anemia, autoimmune hemolytic anemia, aplastic anemia, diamond Blackfan anemia, Aplasia, Pure Red Cell anemia, asthma, inflammatory chest disease, Behcet's syndrome, chronic fatigue syndrome, clostridium difficile toxin, congenital heart block, cystic fibrosis, intractable pediatric epilepsy, juvenile arthritis, myositis, polymyositis, multiple myeloma and immunoproliferative neoplasms, motor neuron syndromes, myasthenia gravis, myelopathy associated with Human T-cell leukemia/lymphoma virus-I, nephrotic syndrome, membraneous neuropathy, paraproteinemic neuropathy, euthyroid opthalmopathy, recurrent otitis media, pemphigus vulgaris, pemphigus foliaceus, paraneoplastic pemphigus, progressive lumbosacral plexopathy, post-transfusion purpura, recurrent fetal loss, renal failure, rheumatoid arthritis, systemic lupus erythematosus and related cytopenia, nephritis, CNS involvement, vasculitis, pericarditis, or pleural effusion, thrombotic thrombocytopenic purpura, nonimmune thrombocytopenia, neonatal alloimmune thrombocytopenia (pre- and postnatal), septic thrombocytopenia, quinine induced thrombocytopenia, transfusion reactions, uveitis, systemic vasculitic syndromes, acquired Von Willebrand's syndrome and others.

Immune Globulin infusions must be carefully prescribed and administered. IVIG infusions are often administered by an infusion protocol whereby the rate of infusion is increased in a step-wise fashion. Prior to each increase in the infusion rate (e.g., each "step up"), the patient is monitored for signs of adverse reaction to the drug infusion. If no adverse reaction is noted and the patient appears to be tolerating the infusion, then the infusion rate is increased (e.g., stepped up). The types of adverse reaction that may occur as a result of IVIG infusion include migraine headache, flushing, nausea, vomiting, chills and others. There is also a risk of more serious, sometimes life-threatening reactions, for example, risk of thrombus formation. Particular care must be given to patients having certain health issues such as a history of stroke, heart attack, blood vessel disease, IgA or IgG deficiencies or blood clots.

Other particular uses for programmable infusion pumps include, but are not limited to, the administration of analgesics, anesthetics, cancer chemotherapy, antibiotics, gene therapy agents, anti-venoms and other drugs or substances that require carefully controlled and/or monitored infusion to avoid harmful reactions, overdosing, allergic responses, anaphylactic responses, other idiosyncratic responses, etc.

As illustrated in the figures, in some embodiments, the infusion pumping devices 22, 26 may incorporate an apparatus for monitoring the patient's bodily or physiologic variables which may indicate or may be predictive of an adverse reaction to the infusion. The user interface devices 20, 24 may access such monitored bodily or physiologic variables and/or may receive alarms when such monitored bodily or physiologic variables are outside of preset limits. The user (e.g., physician, pharmacist, charge nurse, physician assistant, etc.) may then make any desired modifications to the patient's prescription (e.g., infusion rate, infusion profile, etc.) in view of changes in the monitored bodily or physiologic variables.

Also as illustrated in the figures, in some embodiments, the infusion pumping devices 22, 26 may incorporate an apparatus for querying the patient and for receiving the patient's responses to such queries. The query or queries may relate to the absence or presence of symptoms or sensations that may indicate or may be predictive of an adverse reaction to the infusion (e.g., headache, sweating, nausea, shivering, blurred vision, etc.). The user interface devices 20, 24 may access the patient's responses to such queries and/or may receive alarms when the patient's responses to the queries when the patient's responses are outside of preset limits. The user (e.g., physician, pharmacist, charge nurse, physician assistant, etc.) may then make any desired modifications to the patient's prescription (e.g., infusion rate, infusion profile, etc.) in view of changes in the patient's responses to such queries.
Further aspects and embodiments of the present invention are described in the following numbered paragraphs.
[1A] In one aspect, the present invention provides a system for monitoring and/or managing infusion therapy over a network, comprising: at least one infusion pump device to deliver infusion therapy; at least one remotely located user interface device to monitor and control the at least one infusion pump device; and at least one server to which the at least one user interface is connected for monitoring and controlling the at least one infusion pump device, such that the at least one user interface and the at least one pump device communicate via the network through the at least one server.
[2A] In an embodiment, the present invention provides a system according to paragraph [1A], wherein the at least one server comprises: a pump server to provide data between the at least one infusion pump device and the at least one server; a web server to provide user access and control to the at least one user interface; and a database to store the data provided by the pump and the at least one user interface.
[3A] In an embodiment, the present invention provides a system according to paragraph [1A], wherein the network is an intranet network, the Internet, or a combination of an intranet network and the Internet.
[4A] In an embodiment, the present invention provides a system according to paragraph [3A], wherein the intranet network is implemented as a wired or wireless network.
[5A] In an embodiment, the present invention provides a system according to paragraph [1A], wherein the at least one user interface device comprises at least one device selected from the group comprising computers, smart telephones incorporating Internet browser capability, personal digital assistants, mobile telephones and the at least one infusion pump.
[6A] In an embodiment, the present invention provides a system according to paragraph [1A], wherein the at least one user interface device is configured to access information stored in the at least one pump device.
[7A] In an embodiment, the present invention provides a system according to paragraph [6A], wherein the accessible information stored in the at least one pump device is related to server-connected infusion pumps that are undergoing infusion therapy.
[8A] In an embodiment, the present invention provides a system according to paragraph [6A], wherein the at least one user interface device is configured to access information stored in the at least one pump device regarding infusions previously administered.
[9A] In an embodiment, the present invention provides a system according to paragraph [6A], wherein the at least one user interface device is configured to access information stored in the at least one pump device regarding patient history.
[10A] In an embodiment, the present invention provides a system according to paragraph [6A], wherein the user interface device is configured to modify the information stored on the at least one pump to control and manage an infusion therapy currently being administered or scheduled to be administered.
[11A] In an embodiment, the present invention provides a system according to paragraph [1A], wherein the at least one user interface device is located remotely from an administration site.
[12A] In an embodiment, the present invention provides a system according to paragraph [1A], wherein the at least one pump device includes an apparatus configured to monitor a bodily or physiologic variable of a patient receiving infusion therapy, and the at least one user interface is configured to control and/or monitor the bodily or physiologic variable.
[13A] In an embodiment, the present invention provides a system according to paragraph [1A], wherein the at least one pump device includes an apparatus for querying a patient receiving infusion therapy, and the at least one user interface is configured to monitor the patient's response thereto.
[14A] In another aspect, the present invention provides a method for monitoring and/or managing infusion therapy over a network, comprising: monitoring at least one infusion pump device using at least one remotely located user interface device; storing information in the infusion pump device and in at least one server to which the at least one user interface is connected for monitoring and/or controlling the at least one infusion pump device; and managing the at least one infusion pump device using the at least one user interface via the network through the at least one server.
[15A] In an embodiment, the present invention provides a method according to paragraph [14A], which includes configuring the at least one server to store: pump server information to provide data between the at least one infusion pump device and the at least one server; web server information to provide user access and control to the at least one user interface; and the data provided by the pump and the at least one user interface.
[16A] In an embodiment, the present invention provides a method according to paragraph [15A], which includes configuring the at least one user interface device to access information stored in the at least one pump device.
[17A] In an embodiment, the present invention provides a method according to paragraph [15A], which includes storing accessible information in the at least one pump device that is related to server-connected infusion pumps that are undergoing infusion therapy.
[18A] In an embodiment, the present invention provides a method according to paragraph [15A], which includes configuring the at least one user interface device to access information stored in the at least one pump device regarding infusions previously administered.
[19A] In an embodiment, the present invention provides a method according to paragraph [15A], which includes configuring the at least one user interface device to access information stored in the at least one pump device regarding patient history.
[20A] In an embodiment, the present invention provides a method according to paragraph [15A], which includes configuring the user interface device is to modify the information stored on the at least one pump to control and manage an infusion therapy currently being administered or scheduled to be administered.
[21A] In an embodiment, the present invention provides a method according to paragraph [14A], which includes locating the at least one user interface device remotely from an administration site.
[22A] In an embodiment, the present invention provides a system according to paragraph [1A], (i) which includes at least one pump device to monitor a bodily or physiologic variable of a patient receiving infusion therapy, and (ii) the at least one user interface to control and monitor the bodily or physiologic variable.
[23A] In an embodiment, the present invention provides a method according to paragraph [14A], which includes locating the at least one pump device to query a patient receiving infusion therapy, and the at least one user interface to monitor the patient's response thereto.
It is to be appreciated that the invention has been described herein with reference to certain examples or embodiments of the invention but that various additions, deletions, alterations and modifications may be made to those examples and embodiments without departing from the intended spirit and scope of the invention. For example, any element or attribute of one embodiment or example may be incorporated into or used with another embodiment or example, unless otherwise specified of if to do so would render the embodiment or example unsuitable for its intended use. Also, where the steps of a method or process have been described or listed in a particular order, the order of such steps may be changed unless otherwise specified or unless doing so would render the method or process unworkable for its intended purpose. All reasonable additions, deletions, modifications and alterations are to be considered equivalents of the described examples and embodiments and are to be included within the scope of the following claims.

## Claims

1. A system (10) for monitoring and/or managing at least one infusion pump comprising:
an intra-institutional portion (14) including (i) at least one intra-institutional infusion pump (22), (ii) an intra-institutional user interface (20), and (iii) a plurality of servers (18a, 18b, 18c) to which the at least one user interface is operably connected for monitoring and/or controlling the at least one intra-institutional infusion pump, such that the at least one intra-institutional user interface and the at least one intra-institutional infusion pump can communicate via a network through the plurality of servers, the plurality of servers including an infusion pump server (18a) to allow data flow between the at least one intra-institutional infusion pump and the other of the plurality of servers, and a web server (18b) to provide user access and control to the at least one intra-institutional infusion pump at the at least one user interface; and
an extra-institutional portion (16) of the system including (a) at least one extra-institutional user interface device (24) and (b) at least one extra-institutional infusion pump (26), wherein the extra-institutional user interface device and the extra-institutional infusion pump are operably connected to the intra-institutional portion via the Internet

2. The system according to claim 1, wherein the plurality of servers (18a, 18b, 18c) further includes a database server (18c) to store data provided by the at least one intra-institutional infusion pump (22) and the at least one infra-institutional user interface (20).

3. The system according to claim 1, wherein the network is an intranet network, the Internet, or a combination of an intranet network and the Internet.

4. The system according to claim 3, wherein the intranet network is implemented as a wired or wireless network.

5. The system according to claim 1, wherein the at least one intra-institutional user interface (20) and the at least one extra-institutional user interface (24) each include at least one device selected from the group comprising computers, smart telephones incorporating Internet browser capability, personal digital assistants, mobile telephones and the at least one infusion pump.

6. The system according to claim 1, wherein the at least one intra-institutional user interface (20) is configured to access information stored in the at least one infusion pump (22).

7. The system according to claim 6, wherein the information concerns server-connected intra-institutional infusion pumps that are administering therapy.

8. The system according to claim 6, wherein the information concerns medical treatments previously administered.

9. The system according to claim 6, wherein the information concerns patient history.

10. The system according to claim 6, wherein the intra-institutional user interface (20) is configured to modify the information to control and manage a therapy currently being administered or scheduled to be administered.

11. The system according to claim 1, wherein the at least one intra-institutional user interface (20) is located remotely from an administration site.

12. The system according to claim 1, wherein the at least one intra-institutional infusion pump (22) includes an apparatus configured to monitor a bodily or physiologic variable of a patient receiving therapy, and the at least one intra-institutional user interface (20) is configured to display the bodily or physiologic variable.

13. The system according to claim 1, wherein the at least one intra-institutional infusion pump (22) includes an apparatus for querying a patient receiving therapy, and the at least one user interface (20) is configured to accept the patient's response thereto.
